(19) Europäisches Patentamt
European Patent Office
Office européen des brevets

(11) EP 1 259 251 B1

(12) # EUROPEAN PATENT SPECIFICATION

(45) Date of publication and mention of the grant of the patent:
**19.10.2005 Bulletin 2005/42**

(21) Application number: **01905632.4**

(22) Date of filing: **19.02.2001**

(51) Int Cl.[7]: **A61K 38/17**, A61K 39/00, C07K 14/435, C07K 19/00, A61P 25/28

(86) International application number:
**PCT/DK2001/000113**

(87) International publication number:
**WO 2001/062284 (30.08.2001 Gazette 2001/35)**

(54) **NOVEL METHOD FOR DOWN-REGULATION OF AMYLOID**

VERFAHREN ZUR HERABREGULIERUNG VON AMYLOID

NOUVELLE METHODE DE REGULATION NEGATIVE D'AMYLOIDE

(84) Designated Contracting States:
**AT BE CH CY DE DK ES FI FR GB GR IE IT LI LU MC NL PT SE TR**
Designated Extension States:
**AL LT LV MK RO SI**

(30) Priority: **21.02.2000 DK 200000265**
**01.03.2000 US 186295 P**

(43) Date of publication of application:
**27.11.2002 Bulletin 2002/48**

(73) Proprietor: **Pharmexa A/S**
**2970 Horsholm (DK)**

(72) Inventors:
- **BIRK, Peter Pharmexa A/S**
  **DK-2970 Horsholm (DK)**
- **JENSEN, Martin, Roland Pharmexa A/S**
  **DK-2970 Horsholm (DK)**
- **NIELSEN, Klaus, Gregorius Pharmexa A/S**
  **DK-2970 Horsholm (DK)**

(74) Representative: **Inspicos A/S**
**Böge Allé 5,**
**P.O. Box 45**
**2970 Hörsholm (DK)**

(56) References cited:
**WO-A1-95/05849**
**WO-A1-95/23166**
**WO-A1-99/27944**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

SEQUENCE LISTING

<110> M&E Biotech A/S

<120> Novel Method For Down-Regulation Of Amyloid

<130> P1009PC1

<140>
<141>

<160> 16

<170> PatentIn Ver. 3.0

<210> 1
<211> 2313
<212> DNA
<213> Homo sapiens

<220>
<221> CDS
<222> (1)..(2313)

<220>
<221> misc_feature
<222> (2098)..(2169)
<223> nucleotides encoding transmembrane region

<220>
<221> mise_feature
<222> (2019)..(2313)
<223> Nucleotides encoding C-100

<220>
<221> misc_feature
<222> (2016)..(2144)
<223> Abeta 42/43

<220>
<221> misc_feature
<222> (2014)..(2142)
<223> Abeta 42/43
<400> 1

```
atg ctg ccc ggt ttg gca ctg ctc ctg ctg gcc gcc tgg acg gct cgg    48
Met Leu Pro Gly Leu Ala Leu Leu Leu Leu Ala Ala Trp Thr Ala Arg
 1               5                  10                  15

gcg ctg gag gta ccc act gat ggt aat gct ggc ctg ctg gct gaa ccc    96
Ala Leu Glu Val Pro Thr Asp Gly Asn Ala Gly Leu Leu Ala Glu Pro
            20                  25                  30

cag att gcc atg ttc tgt ggc aga ctg aac atg cac atg aat gtc cag   144
Gln Ile Ala Met Phe Cys Gly Arg Leu Asn Met His Met Asn Val Gln
        35                  40                  45

aat ggg aag tgg gat tca gat cca tca ggg acc aaa acc tgc att gat   192
Asn Gly Lys Trp Asp Ser Asp Pro Ser Gly Thr Lys Thr Cys Ile Asp
    50                  55                  60

acc aag gaa ggc atc ctg cag tat tgc caa gaa gtc tac cct gaa ctg   240
Thr Lys Glu Gly Ile Leu Gln Tyr Cys Gln Glu Val Tyr Pro Glu Leu
65                  70                  75                  80

cag atc acc aat gtg gta gaa gcc aac caa cca gtg acc atc cag aac   288
Gln Ile Thr Asn Val Val Glu Ala Asn Gln Pro Val Thr Ile Gln Asn
                85                  90                  95

tgg tgc aag cgg ggc cgc aag cag tgc aag acc cat ccc cac ttt gtg   336
Trp Cys Lys Arg Gly Arg Lys Gln Cys Lys Thr His Pro His Phe Val
            100                 105                 110

att ccc tac cgc tgc tta gtt ggt gag ttt gta agt gat gcc ctt ctc   384
Ile Pro Tyr Arg Cys Leu Val Gly Glu Phe Val Ser Asp Ala Leu Leu
        115                 120                 125

gtt cct gac aag tgc aaa ttc tta cac cag gag agg atg gat gtt tgc   432
Val Pro Asp Lys Cys Lys Phe Leu His Gln Glu Arg Met Asp Val Cys
    130                 135                 140
```

```
gaa act cat ctt cac tgg cac acc gtc gcc aaa gag aca tgc agt gag   480
Glu Thr His Leu His Trp His Thr Val Ala Lys Glu Thr Cys Ser Glu
145                 150                 155                 160

aag agt acc aac ttg cat gac tac ggc atg ttg ctg ccc tgc gga att   528
Lys Ser Thr Asn Leu His Asp Tyr Gly Met Leu Leu Pro Cys Gly Ile
                165                 170                 175

gac aag ttc cga ggg gta gag ttt gtg tgt tgc cca ctg gct gaa gaa   576
Asp Lys Phe Arg Gly Val Glu Phe Val Cys Cys Pro Leu Ala Glu Glu
            180                 185                 190

agt gac aat gtg gat tct gct gat gcg gag gag gat gac tcg gat gtc   624
Ser Asp Asn Val Asp Ser Ala Asp Ala Glu Glu Asp Asp Ser Asp Val
        195                 200                 205

tgg tgg ggc gga gca gac aca gac tat gca gat ggg agt gaa gac aaa   672
Trp Trp Gly Gly Ala Asp Thr Asp Tyr Ala Asp Gly Ser Glu Asp Lys
    210                 215                 220

gta gta gaa gta gca gag gag gaa gaa gtg gct gag gtg gaa gaa gaa   720
Val Val Glu Val Ala Glu Glu Glu Glu Val Ala Glu Val Glu Glu Glu
225                 230                 235                 240

gaa gcc gat gat gac gag gac gat gag gat ggt gat gag gta gag gaa   768
Glu Ala Asp Asp Asp Glu Asp Asp Glu Asp Gly Asp Glu Val Glu Glu
                245                 250                 255

gag gct gag gaa ccc tac gaa gaa gcc aca gag aga acc acc agc att   816
Glu Ala Glu Glu Pro Tyr Glu Glu Ala Thr Glu Arg Thr Thr Ser Ile
            260                 265                 270

gcc acc acc acc acc acc acc aca gag tct gtg gaa gag gtg gtt cga   864
Ala Thr Thr Thr Thr Thr Thr Thr Glu Ser Val Glu Glu Val Val Arg
        275                 280                 285

gag gtg tgc tct gaa caa gcc gag acg ggg ccg tgc cga gca atg atc   912
Glu Val Cys Ser Glu Gln Ala Glu Thr Gly Pro Cys Arg Ala Met Ile
    290                 295                 300
```

tcc cgc tgg tac ttt gat gtg act gaa ggg aag tgt gcc cca ttc ttt 960
Ser Arg Trp Tyr Phe Asp Val Thr Glu Gly Lys Cys Ala Pro Phe Phe
305 310 315 320

tac ggc gga tgt ggc ggc aac cgg aac aac ttt gac aca gaa gag tac 1008
Tyr Gly Gly Cys Gly Gly Asn Arg Asn Asn Phe Asp Thr Glu Glu Tyr
325 330 335

tgc atg gcc gtg tgt ggc agc gcc atg tcc caa agt tta ctc aag act 1056
Cys Met Ala Val Cys Gly Ser Ala Met Ser Gln Ser Leu Leu Lys Thr
340 345 350

acc cag gaa cct ctt gcc cga gat cct gtt aaa ctt cct aca aca gca 1104
Thr Gln Glu Pro Leu Ala Arg Asp Pro Val Lys Leu Pro Thr Thr Ala
355 360 365

gcc agt acc cct gat gcc gtt gac aag tat ctc gag aca cct ggg gat 1152
Ala Ser Thr Pro Asp Ala Val Asp Lys Tyr Leu Glu Thr Pro Gly Asp
370 375 380

gag aat gaa cat gcc cat ttc cag aaa gcc aaa gag agg ctt gag gcc 1200
Glu Asn Glu His Ala His Phe Gln Lys Ala Lys Glu Arg Leu Glu Ala
385 390 395 400

aag cac cga gag aga atg tcc cag gtc atg aga gaa tgg gaa gag gca 1248
Lys His Arg Glu Arg Met Ser Gln Val Met Arg Glu Trp Glu Glu Ala
405 410 415

gaa cgt caa gca aag aac ttg cct aaa gct gat aag aag gca gtt atc 1296
Glu Arg Gln Ala Lys Asn Leu Pro Lys Ala Asp Lys Lys Ala Val Ile
420 425 430

cag cat ttc cag gag aaa gtg gaa tct ttg gaa cag gaa gca gcc aac 1344
Gln His Phe Gln Glu Lys Val Glu Ser Leu Glu Gln Glu Ala Ala Asn
435 440 445

gag aga cag cag ctg gtg gag aca cac atg gcc aga gtg gaa gcc atg 1392
Glu Arg Gln Gln Leu Val Glu Thr His Met Ala Arg Val Glu Ala Met
450 455 460

```
ctc aat gac cgc cgc cgc ctg gcc ctg gag aac tac atc acc gct ctg   1440
Leu Asn Asp Arg Arg Arg Leu Ala Leu Glu Asn Tyr Ile Thr Ala Leu
465                 470                 475                 480

cag gct gtt cct cct cgg cct cgt cac gtg ttc aat atg cta aag aag   1488
Gln Ala Val Pro Pro Arg Pro Arg His Val Phe Asn Met Leu Lys Lys
                485                 490                 495

tat gtc cgc gca gaa cag aag gac aga cag cac acc cta aag cat ttc   1536
Tyr Val Arg Ala Glu Gln Lys Asp Arg Gln His Thr Leu Lys His Phe
            500                 505                 510

gag cat gtg cgc atg gtg gat ccc aag aaa gcc gct cag atc cgg tcc   1584
Glu His Val Arg Met Val Asp Pro Lys Lys Ala Ala Gln Ile Arg Ser
        515                 520                 525

cag gtt atg aca cac ctc cgt gtg att tat gag cgc atg aat cag tct   1632
Gln Val Met Thr His Leu Arg Val Ile Tyr Glu Arg Met Asn Gln Ser
    530                 535                 540

ctc tcc ctg ctc tac aac gtg cct gca gtg gcc gag gag att cag gat   1680
Leu Ser Leu Leu Tyr Asn Val Pro Ala Val Ala Glu Glu Ile Gln Asp
545                 550                 555                 560

gaa gtt gat gag ctg ctt cag aaa gag caa aac tat tca gat gac gtc   1728
Glu Val Asp Glu Leu Leu Gln Lys Glu Gln Asn Tyr Ser Asp Asp Val
                565                 570                 575

ttg gcc aac atg att agt gaa cca agg atc agt tac gga aac gat gct   1776
Leu Ala Asn Met Ile Ser Glu Pro Arg Ile Ser Tyr Gly Asn Asp Ala
            580                 585                 590

ctc atg cca tct ttg acc gaa acg aaa acc acc gtg gag ctc ctt ccc   1824
Leu Met Pro Ser Leu Thr Glu Thr Lys Thr Thr Val Glu Leu Leu Pro
        595                 600                 605

gtg aat gga gag ttc agc ctg gac gat ctc cag ccg tgg cat tct ttt   1872
Val Asn Gly Glu Phe Ser Leu Asp Asp Leu Gln Pro Trp His Ser Phe
    610                 615                 620
```

```
ggg gct gac tct gtg cca gcc aac aca gaa aac gaa gtt gag cct gtt      1920
Gly Ala Asp Ser Val Pro Ala Asn Thr Glu Asn Glu Val Glu Pro Val
625                 630                 635                 640

gat gcc cgc cct gct gcc gac cga gga ctg acc act cga cca ggt tct      1968
Asp Ala Arg Pro Ala Ala Asp Arg Gly Leu Thr Thr Arg Pro Gly Ser
                645                 650                 655

ggg ttg aca aat atc aag acg gag gag atc tct gaa gtg aag atg gat      2016
Gly Leu Thr Asn Ile Lys Thr Glu Glu Ile Ser Glu Val Lys Met Asp
            660                 665                 670

gca gaa ttc cga cat gac tca gga tat gaa gtt cat cat caa aaa ttg      2064
Ala Glu Phe Arg His Asp Ser Gly Tyr Glu Val His His Gln Lys Leu
        675                 680                 685

gtg ttc ttt gca gaa gat gtg ggt tca aac aaa ggt gca atc att gga      2112
Val Phe Phe Ala Glu Asp Val Gly Ser Asn Lys Gly Ala Ile Ile Gly
    690                 695                 700

ctc atg gtg ggc ggt gtt gtc ata gcg aca gtg atc gtc atc acc ttg      2160
Leu Met Val Gly Gly Val Val Ile Ala Thr Val Ile Val Ile Thr Leu
705                 710                 715                 720

gtg atg ctg aag aag aaa cag tac aca tcc att cat cat ggt gtg gtg      2208
Val Met Leu Lys Lys Lys Gln Tyr Thr Ser Ile His His Gly Val Val
                725                 730                 735

gag gtt gac gcc gct gtc acc cca gag gag cgc cac ctg tcc aag atg      2256
Glu Val Asp Ala Ala Val Thr Pro Glu Glu Arg His Leu Ser Lys Met
            740                 745                 750

cag cag aac ggc tac gaa aat cca acc tac aag ttc ttt gag cag atg      2304
Gln Gln Asn Gly Tyr Glu Asn Pro Thr Tyr Lys Phe Phe Glu Gln Met
        755                 760                 765

cag aac tag                                                          2313
Gln Asn
    770
```

<210> 2

<211> 770
<212> PRT
<213> Homo sapiens

<400> 2

```
Met Leu Pro Gly Leu Ala Leu Leu Leu Leu Ala Ala Trp Thr Ala Arg
1               5                   10                  15

Ala Leu Glu Val Pro Thr Asp Gly Asn Ala Gly Leu Leu Ala Glu Pro
            20                  25                  30

Gln Ile Ala Met Phe Cys Gly Arg Leu Asn Met His Met Asn Val Gln
        35                  40                  45

Asn Gly Lys Trp Asp Ser Asp Pro Ser Gly Thr Lys Thr Cys Ile Asp
    50                  55                  60

Thr Lys Glu Gly Ile Leu Gln Tyr Cys Gln Glu Val Tyr Pro Glu Leu
65                  70                  75                  80

Gln Ile Thr Asn Val Val Glu Ala Asn Gln Pro Val Thr Ile Gln Asn
                85                  90                  95

Trp Cys Lys Arg Gly Arg Lys Gln Cys Lys Thr His Pro His Phe Val
            100                 105                 110

Ile Pro Tyr Arg Cys Leu Val Gly Glu Phe Val Ser Asp Ala Leu Leu
        115                 120                 125

Val Pro Asp Lys Cys Lys Phe Leu His Gln Glu Arg Met Asp Val Cys
    130                 135                 140

Glu Thr His Leu His Trp His Thr Val Ala Lys Glu Thr Cys Ser Glu
145                 150                 155                 160

Lys Ser Thr Asn Leu His Asp Tyr Gly Met Leu Leu Pro Cys Gly Ile
                165                 170                 175
```

Asp Lys Phe Arg Gly Val Glu Phe Val Cys Cys Pro Leu Ala Glu Glu
180 185 190

Ser Asp Asn Val Asp Ser Ala Asp Ala Glu Glu Asp Asp Ser Asp Val
195 200 205

Trp Trp Gly Gly Ala Asp Thr Asp Tyr Ala Asp Gly Ser Glu Asp Lys
210 215 220

Val Val Glu Val Ala Glu Glu Glu Glu Val Ala Glu Val Glu Glu Glu
225 230 235 240

Glu Ala Asp Asp Asp Glu Asp Asp Glu Asp Gly Asp Glu Val Glu Glu
245 250 255

Glu Ala Glu Glu Pro Tyr Glu Glu Ala Thr Glu Arg Thr Thr Ser Ile
260 265 270

Ala Thr Thr Thr Thr Thr Thr Thr Glu Ser Val Glu Glu Val Val Arg
275 280 285

Glu Val Cys Ser Glu Gln Ala Glu Thr Gly Pro Cys Arg Ala Met Ile
290 295 300

Ser Arg Trp Tyr Phe Asp Val Thr Glu Gly Lys Cys Ala Pro Phe Phe
305 310 315 320

Tyr Gly Gly Cys Gly Gly Asn Arg Asn Asn Phe Asp Thr Glu Glu Tyr
325 330 335

Cys Met Ala Val Cys Gly Ser Ala Met Ser Gln Ser Leu Leu Lys Thr
340 345 350

Thr Gln Glu Pro Leu Ala Arg Asp Pro Val Lys Leu Pro Thr Thr Ala
355 360 365

Ala Ser Thr Pro Asp Ala Val Asp Lys Tyr Leu Glu Thr Pro Gly Asp
370 375 380

Glu Asn Glu His Ala His Phe Gln Lys Ala Lys Glu Arg Leu Glu Ala
385 390 395 400

Lys His Arg Glu Arg Met Ser Gln Val Met Arg Glu Trp Glu Glu Ala
405 410 415

Glu Arg Gln Ala Lys Asn Leu Pro Lys Ala Asp Lys Lys Ala Val Ile
420 425 430

Gln His Phe Gln Glu Lys Val Glu Ser Leu Glu Gln Glu Ala Ala Asn
435 440 445

Glu Arg Gln Gln Leu Val Glu Thr His Met Ala Arg Val Glu Ala Met
450 455 460

Leu Asn Asp Arg Arg Arg Leu Ala Leu Glu Asn Tyr Ile Thr Ala Leu
465 470 475 480

Gln Ala Val Pro Pro Arg Pro Arg His Val Phe Asn Met Leu Lys Lys
485 490 495

Tyr Val Arg Ala Glu Gln Lys Asp Arg Gln His Thr Leu Lys His Phe
500 505 510

Glu His Val Arg Met Val Asp Pro Lys Lys Ala Ala Gln Ile Arg Ser
515 520 525

Gln Val Met Thr His Leu Arg Val Ile Tyr Glu Arg Met Asn Gln Ser
530 535 540

Leu Ser Leu Leu Tyr Asn Val Pro Ala Val Ala Glu Glu Ile Gln Asp
545 550 555 560

Glu Val Asp Glu Leu Leu Gln Lys Glu Gln Asn Tyr Ser Asp Asp Val
565 570 575

Leu Ala Asn Met Ile Ser Glu Pro Arg Ile Ser Tyr Gly Asn Asp Ala
580 585 590

Leu Met Pro Ser Leu Thr Glu Thr Lys Thr Thr Val Glu Leu Leu Pro
595 600 605

Val Asn Gly Glu Phe Ser Leu Asp Asp Leu Gln Pro Trp His Ser Phe
610 615 620

Gly Ala Asp Ser Val Pro Ala Asn Thr Glu Asn Glu Val Glu Pro Val
625 630 635 640

Asp Ala Arg Pro Ala Ala Asp Arg Gly Leu Thr Thr Arg Pro Gly Ser
645 650 655

Gly Leu Thr Asn Ile Lys Thr Glu Glu Ile Ser Glu Val Lys Met Asp
660 665 670

Ala Glu Phe Arg His Asp Ser Gly Tyr Glu Val His His Gln Lys Leu
675 680 685

Val Phe Phe Ala Glu Asp Val Gly Ser Asn Lys Gly Ala Ile Ile Gly
690 695 700

Leu Met Val Gly Gly Val Val Ile Ala Thr Val Ile Val Ile Thr Leu
705 710 715 720

Val Met Leu Lys Lys Lys Gln Tyr Thr Ser Ile His His Gly Val Val
725 730 735

Glu Val Asp Ala Ala Val Thr Pro Glu Glu Arg His Leu Ser Lys Met
740 745 750

Gln Gln Asn Gly Tyr Glu Asn Pro Thr Tyr Lys Phe Phe Glu Gln Met
755 760 765

Gln Asn
770

<210> 3
<211> 45
<212> DNA
<213> Clostridium tetani

<220>
<221> CDS
<222> (1)..(45)
<223> DNA encoding P2 epitope

<400> 3

```
cag tac atc aaa gct aac tcc aaa ttc atc ggt atc acc gag ctg       45
Gln Tyr Ile Lys Ala Asn Ser Lys Phe Ile Gly Ile Thr Glu Leu
 1               5                   10                  15
```

<210> 4
<211> 15
<212> PRT
<213> Clostridium tetani

<400> 4

```
Gln Tyr Ile Lys Ala Asn Ser Lys Phe Ile Gly Ile Thr Glu Leu
 1               5                   10                  15
```

<210> 5
<211> 63
<212> DNA
<213> Clostridium tetani

<220>
<221> CDS
<222> (1)..(63)
<223> DNA encoding P30 epitope

<400> 5

```
ttc aac aac ttc acc gta agc ttc tgg ctg cgt gtt ccg aaa gtt agc   48
Phe Asn Asn Phe Thr Val Ser Phe Trp Leu Arg Val Pro Lys Val Ser
 1               5                   10                  15

gct agc cac ctg gaa                                                63
Ala Ser His Leu Glu
            20
```

<210> 6
<211> 21
<212> PRT

<213> Clostridium tetani

<400> 6

Phe Asn Asn Phe Thr Val Ser Phe Trp Leu Arg Val Pro Lys Val Ser
1 5 10 15

Ala Ser His Leu Glu
20

<210> 7
<211> 21
<212> DNA
<213> Synthetic

<400> 7

caactcagct tcctttcggg c 21

<210> 8
<211> 21
<212> DNA
<213> Synthetic

<400> 8

agatctcgat cccgcgaaat t 21

<210> 9
<211> 135
<212> DNA
<213> Synthetic

<400> 9

atggatgcag aattccgtca cgactccggt tacgaagttc accaccagaa actggttttc 60

ttcgcagaag atgttggttc caacaaaggt gcaatcatcg gtctgatggt tggcggtgtt 120
gttatcgcga cctag 135

<210> 10
<211> 31
<212> DNA
<213> Synthetic

<400> 10

gccggccatg gatgcagaat tccgtcacga c 31

<210> 11
<211> 39
<212> DNA
<213> Synthetic

<400> 11

gccggaagct tctaggtcgc gataacaaca ccgccaacc 39

<210> 12
<211> 84
<212> DNA
<213> Synthetic

<400> 12

ccggcaagct tctacagctc ggtgataccg atgaatttgg agttagcttt gatgtactgg 60
gtcgcgataa caacaccgcc aacc 84

<210> 13
<211> 101
<212> DNA
<213> Synthetic

<400> 13

gccggccatg ggtttcaaca acttcaccgt tagcttctgg ctgcgtgttc cgaaagttag 60
cgcgagccac ctggaagatg cagaattccg tcacgactcc g 101

<210> 14
<211> 172
<212> DNA
<213> Synthetic

<400> 14

gggccaagct tggatccggt cgcgataaca acaccgccaa ccatcagacc gatgattgca 60
cctttgttgg aaccaacatc ttctgcgaag aaaaccagtt tctggtggtg aacttcgtaa 120
ccggagtcgt gacggaactc tgcatccagc tcggtgatac cgatgaattt gg 172

<210> 15
<211> 30
<212> DMA
<213> Synthetic

<400> 15

ctggaagatg cagagttccg tcacgactcc 30

<210> 16
<211> 35
<212> DNA
<213> Synthetic

<400> 16

gcgccggatc cttcaacaac ttcaccgtta gcttc 35

## Description

FIELD OF THE INVENTION

The present invention relates to improvements in therapy and prevention of Alzheimer's disease (AD) and other diseases characterized by deposition of amyloid, e.g. characterized by amyloid deposits in the central nervous system (CNS). More specifically, the present invention enables a method for down-regulating (undesired) deposits of amyloid by enabling the production of antibodies against the relevant protein or components thereof in subjects suffering from or in danger of suffering from diseases having a pathology involving amyloid deposition. The invention also provides for methods of producing polypeptides useful in this method as well as for the modified polypeptides as such. Also encompassed by the present invention are nucleic acid fragments encoding the modified polypeptides as well as vectors incorporating these nucleic acid fragments and host cells and cell lines transformed therewith. The invention also provides for compositions comprising modified polypeptides or comprising nucleic acids encoding modified polypeptides.

BACKGROUND OF THE INVENTION

Amyloidosis is the extracellular deposition of insoluble protein fibrils leading to tissue damage and disease (Pepys, 1996; Tan et al., 1995; Kelly, 1996). The fibrils form when normally soluble proteins and peptides self-associate in an abnormal manner (Kelly, 1997).
Amyloid is associated with serious diseases including systemic amyloidosis, AD, maturity onset diabetes, Parkinson's disease, Huntington's disease, fronto-temporal dementia and the prion-related transmissible spongiform encephalopathies (kuru and Creutzfeldt-Jacob disease in humans and scrapie and BSE in sheep and cattle, respectively) and the amyloid plaque formation in for instance Alzheimer's seems to be closely associated with the progression of human disease. In animal models over-expression, or the expression of modified forms, of proteins found in deposits, like the β-amyloid protein, has been shown to induce various symptoms of disease, e.g. Alzheimer's-like symptoms. There is no specific treatment for amyloid deposition and these diseases are usually fatal.
The subunits of amyloid fibrils may be wild-type, variant or truncated proteins, and similar fibrils can be formed in vitro from oligopeptides and denatured proteins (Bradbury et al., 1960; Filshie et al., 1964; Burke & Rougvie, 1972). The nature of the polypeptide component of the fibrils defines the character of the amyloidosis. Despite large differences in the size, native structure and function of amyloid proteins, all amyloid fibrils are of indeterminate length, unbranched, 70 to 120 Å in diameter, and display characteristic staining with Congo Red (Pepys, 1996). They are characteristic of a cross-β structure (Pauling & Corey, 1951) in which the polypeptide chain is organized in β-sheets. Although the amyloid proteins have very different precursor structures, they can all undergo a structural conversion, perhaps along a similar pathway, to a misfolded form that is the building block of the β-sheet helix protofilament.
This distinctive fibre pattern led to the amyloidoses being called the β-fibrilloses (Glenner, 1980a,b), and the fibril protein of AD was named the β-protein before its secondary structure was known (Glenner & Wong, 1984). The characteristic cross-β diffraction pattern, together with the fibril appearance and tinctorial properties are now the accepted diagnostic hallmarks of amyloid, and suggest that the fibrils, although formed from quite different protein precursors, share a degree of structural similarity and comprise a structural superfamily, irrespective of the nature of their precursor proteins (Sunde M, Serpell LC, Bartlam M, Fraser PE, Pepys MB, Blake CCFJ Mol Biol 1997 Oct 31; 273 (3):729-739).
One of the most widespread and well-known diseases where amyloid deposits in the central nervus system are suggested to have a central role in the progression of the disease, is AD.

### AD

Alzheimer's disease (AD) is an irreversible, progressive brain disorder that occurs gradually and results in memory loss, behavioural and personality changes, and a decline in mental abilities. These losses are related to the death of brain cells and the breakdown of the connections between them. The course of this disease varies from person to person, as does the rate of decline. On average, AD patients live for 8 to 10 years after they are diagnosed, though the disease can last for up to 20 years.
AD advances by stages, from early, mild forgetfulness to a severe loss of mental function. This loss is known as dementia. In most people with AD, symptoms first appear after the age of 60, but earlier onsets are not infrequent. The earliest symptoms often include loss of recent memory, faulty judgment, and changes in personality. Often, people in the initial stages of AD think less clearly and forget the names of familiar people and common objects. Later in the disease, they may forget how to do even simple tasks. Eventually, people with AD lose all reasoning ability and become dependent on other people for their everyday care. Ultimately, the disease becomes so debilitating that patients are bedridden and likely to develop other illnesses and infections. Most commonly, people with AD die from pneumonia.

Although the risk of developing AD increases with age, AD and dementia symptoms are not a part of normal aging. AD and other dementing disorders are caused by diseases that affect the brain. In normal aging, nerve cells in the brain are not lost in large numbers. In contrast, AD disrupts three key processes: Nerve cell communication, metabolism, and repair. This disruption ultimately causes many nerve cells to stop functioning, lose connections with other nerve cells, and die.

At first, AD destroys neurons in parts of the brain that control memory, especially in the hippocampus and related structures. As nerve cells in the hippocampus stop functioning properly, short-term memory fails, and often, a person's ability to do easy and familiar tasks begins to decline. AD also attacks the cerebral cortex, particularly the areas responsible for language and reasoning. Eventually, many other areas of the brain are involved, all these brain regions atrophy (shrink), and the AD patient becomes bedridden, incontinent, totally helpless, and unresponsive to the outside world (source: National Institute on Aging Progress Report on Alzheimer's Disease, 1999).

**The Impact of AD**

AD is the most common cause of dementia among people age 65 and older. It presents a major health problem because of its enormous impact on individuals, families, the health care system, and society as a whole. Scientists estimate that up to 4 million people currently suffer from the disease, and the prevalence doubles every 5 years beyond age 65. It is also estimated that approximately 360,000 new cases (incidence) will occur each year, though this number will increase as the population ages (Brookmeyer *et al*., 1998).

AD puts a heavy economic burden on society. A recent study in the United States estimated that the annual cost of caring for one AD patient is $18,408 for a patient with mild AD, $30,096 for a patient with moderate AD, and $36,132 for a patient with severe AD. The annual national cost of caring for AD patients in the US is estimated to be slightly over $50 billion (Leon *et al*., 1998).

Approximately 4 million Americans are 85 or older, and in most industrialized countries, this age group is one of the fastest growing segments of the population. It is estimated that this group will number nearly 8.5 million by the year 2030 in the US; some experts who study population trends suggest that the number could be even greater. As more and more people live longer, the number of people affected by diseases of aging, including AD, will continue to grow. For example, some studies show that nearly half of all people age 85 and older have some form of dementia. (National Institute on Aging Progress Report on Alzheimer's Disease, 1999)

**The Main Characteristics of AD**

Two abnormal structures in the brain are the hallmarks of AD: amyloid plaques and neurofibrillary tangles (NFT). Plaques are dense, largely insoluble deposits of protein and cellular material outside and around the brain's neurons. Tangles are insoluble twisted fibres that build up inside neurons.

Two types of AD exist: familial AD (FAD), which follows a certain pattern of inheritance, and sporadic AD, where no obvious pattern of inheritance is seen. Because of differences in the age at onset, AD is further described as early-onset (occurring in people younger than 65) or late-onset (occurring in those 65 and older). Early-onset AD is rare (about 10 percent of cases) and generally affects people aged 30 to 60. Some forms of early-onset AD are inherited and run in families. Early-onset AD also often progresses faster than the more common, late-onset form.

All FADs known so far have an early onset, and as many as 50 percent of FAD cases are now known to be caused by defects in three genes located on three different chromosomes. These are mutations in the APP gene on chromosome 21; mutations in a gene on chromosome 14, called presenilin 1; and mutations in a gene on chromosome 1, called presenilin 2. There is as yet no evidence, however, that any of these mutations play a major role in the more common, sporadic or non-familial form of late-onset AD. (National Institute on Aging Progress Report on Alzheimer's Disease, 1999)

**Amyloid Plaques**

In AD, amyloid plaques develop first in areas of the brain used for memory and other cognitive functions. They consist of largely insoluble deposits of beta amyloid (hereinafter designated Aβ) - a protein fragment of a larger protein called amyloid precursor protein (APP, the amino acid sequence of which is set forth in SEQ ID NO: 2) - intermingled with portions of neurons and with non-nerve cells such as microglia and astrocytes. It is not known whether amyloid plaques themselves constitute the main cause of AD or whether they are a by-product of the AD process. Certainly, changes in the APP protein can cause AD, as shown in the inherited form of AD caused by mutations in the APP gene, and Aβ plaque formation seems to be closely associated with the progression of the human disease (Lippa C. F. *et al*. 1998).

## APP

APP is one of many proteins that are associated with cell membranes. After it is made, APP becomes embedded in the nerve cell's membrane, partly inside and partly outside the cell. Recent studies using transgenic mice demonstrate that APP appears to play an important role in the growth and survival of neurons. For example, certain forms and amounts of APP may protect neurons against both short- and long-term damage and may render damaged neurons better able to repair themselves and help parts of neurons grow after brain injury.

While APP is embedded in the cell membrane, proteases act on particular sites in APP, cleaving it into protein fragments. One protease helps cleave APP to form Aβ, and another protease cleaves APP in the middle of the amyloid fragment so that Aβ cannot be formed. The Aβ formed is of two different lengths, a shorter 40 (or 41) amino acids Aβ that is relatively soluble and aggregates slowly, and a slightly longer, 42 amino acids "sticky" Aβ that rapidly forms insoluble clumps. While Aβ is being formed, it is not yet known exactly how it moves through or around nerve cells. In the final stages of this process, the "sticky" Aβ aggregates into long filaments outside the cell and, along with fragments of dead and dying neurons and the microglia and astrocytes, forms the plaques that are characteristic of AD in brain tissue.

Some evidence exists that the mutations in APP render more likely that Aβ will be snipped out of the APP precursor, thus causing either more total Aβ or relatively more of the "sticky" form to be made. It also appears that mutations in the presenilin genes may contribute to the degeneration of neurons in at least two ways: By modifying Aβ production or by triggering the death of cells more directly. Other researchers suggest that mutated presenilins 1 and 2 may be involved in accelerating the pace of apoptosis.

It is to be expected that as the disease progresses, more and more plaques will be formed, filling more and more of the brain. Studies suggest that it may be that the Aβ is aggregating and disaggregating at the same time, in a sort of dynamic equilibrium. This raises the hope that it may be possible to break down the plaques even after they have formed. (National Institute on Aging Progress Report on Alzheimer's Disease, 1999).

It is believed that Aβ is toxic to neurons. In tissue culture studies, researchers observed an increase in death of hippocampal neurons cells engineered to over-express mutated forms of human APP compared to neurons over-expressing the normal human APP (Luo *et al*., 1999).

Furthermore, overexpression or the expression of modified forms of the Aβ protein has in animal models been demonstrated to induce Alzheimer-like symptoms, (Hsiao K. et al., 1998)

Given that increased Aβ generation, its aggregation into plaques, and the resulting neurotoxicity may lead to AD, it is of therapeutic interest to investigate conditions under which Aβ aggregation into plaques might be slowed down or even blocked.

## Presenilins

Mutations in presenilin-1 (S-180) account for almost 50% of all cases of early-onset familial AD (FAD). Around 30 mutations have been identified that give rise to AD. The onset of AD varies with the mutations. Mutations in presenilin-2 account for a much smaller part of the cases of FAD, but is still a significant factor. It is not known whether presenilins are involved in sporadic non-familial AD. The function of the presenilins is not known, but they appear to be involved in the processing of APP to give Aβ-42 (the longer stickier form of the peptide, SEQ ID NO: 2, residues 673-714), since AD patients with presenilin mutations have increased levels of this peptide. It is unclear whether the presenilins also have a role in causing the generation of NFT's. Some suggest that presenilins could also have a more direct role in the degeneration of neurons and neuron death. Presenilin-1 is located at chromosome 14 while presenilin-2 is linked to chromosome 1. If a person harbours a mutated version of just one of these genes he or she is almost certain to develop early onset AD.

There is some uncertainty to whether presenilin-1 is identical to the hypothetical gamma-secretase involved in the processing of APP (Naruse et al., 1998).

## Apolipoprotein E

Apolipoprotein E is usually associated with cholesterol, but is also found in plaques and tangles of AD brains. While alleles 1-3 do not seem to be involved in AD there is a significant correlation between the presence of the APOE-e4 allele and development of late AD (Strittmatter et al., 1993). It is, however, a risk factor and not a direct cause as is the case for the presenilin and APP mutations and it is not limited to familial AD.

The ways in which the ApoE e4 protein increases the likelihood of developing AD are not known with certainty, but one possible theory is that it facilitates Aβ buildup and this contributes to lowering the age of onset of AD, or the presence or absence of particular APOE alleles may affect the way neurons respond to injury (Buttini et al., 1999).

Also Apo A1 has been shown to be amyloigenic. Intact apo A1 can itself form amyloid-like fibrils *in vitro* that are Congo red positive (Am J Pathol 147 (2): 238-244 (Aug 1995), Wisniewski T, Golabek AA, Kida E, Wisniewski KE, Frangione B).

There seem to be some contradictory results indicating that there is a positive effect of the APOE-e4 allele in decreasing symptoms of mental loss, compared to other alleles (Stern, Brandt, 1997, Annals of Neurology 41).

### Neurofibrillary Tangles

This second hallmark of AD consists of abnormal collections of twisted threads found inside nerve cells. The chief component of tangles is one form of a protein called tau (t). In the central nervous system, tau proteins are best known for their ability to bind and help stabilize microtubules, which are one constituent of the cell's internal support structure, or skeleton. However, in AD tau is changed chemically, and this altered tau can no longer stabilize the microtubules, causing them to fall disintegrate. This collapse of the transport system may at first result in malfunctions in communication between nerve cells and may later lead to neuronal death.

In AD, chemically altered tau twists into paired helical filaments - two threads of tau that are wound around each other. These filaments are the major substance found in neurofibrillary tangles. In one recent study, researchers found neurofibrillary changes in fewer than 6 percent of the neurons in a particular part of the hippocampus in healthy brains, in more than 43 percent of these neurons in people who died with mild AD, and in 71 percent of these neurons in people who died with severe AD. When the loss of neurons was studied, a similar progression was found. Evidence of this type supports the idea that the formation of tangles and the loss of neurons progress together over the course of AD. (National Institute on Aging Progress Report on Alzheimer's Disease, 1999).

### Tauopathies and Tangles

Several neurodegenerative diseases, other than AD, are characterized by the aggregation of tau into insoluble filaments in neurons and glia, leading to dysfunction and death. Very recently, several groups of researchers, who were studying families with a variety of hereditary dementias other than AD, found the first mutations in the tau gene on chromosome 17 (Clark *et al*., 1998; Hutton *et al*., 1998; Poorkaj *et al*., 1998; Spillantini *et al*., 1998). In these families, mutations in the tau gene cause neuronal cell death and dementia. These disorders which share some characteristics with AD but differ in several important respects, are collectively called "fronto temporal dementia and parkinsonism linked to chromosome 17" (FTDP-17). They are diseases such as Parkinson's disease, some forms of amyotrophic lateral sclerosis (ALS), corticobasal degeneration, progressive supranuclear palsy, and Pick's disease, and are all characterized by abnormal aggregation of tau protein.

### Other AD-like neurological diseases.

There are important parallels between AD and other neurological diseases, including prion diseases (such as kuru, Creutzfeld-Jacob disease and bovine spongiform encephalitis), Parkinson's disease, Huntington's disease, and fronto-temporal dementia. All involve deposits of abnormal proteins in the brain. AD and prion diseases cause dementia and death, and both are associated with the formation of insoluble amyloid fibrils, but from membrane proteins that are different from each other.

Scientists studying Parkinson's disease, the second most common neurodegenerative disorder after AD, discovered the first gene linked to the disease. This gene codes for a protein called synuclein, which, intriguingly, is also found in the amyloid plaques of AD patients' brains (Lavedan C, 1998, Genome Res. 8(9): 871-80). Investigators have also discovered that genetic defects in Huntington's disease, another progressive neurodegenerative disorder that causes dementia, cause the Huntington protein to form into insoluble fibrils very similar to the Aβ fibrils of AD and the protein fibrils of prion disease, (Scherzinger E, *et al*., 1999, PNAS U.S.A. 96(8): 4604-9).

Scientists have also discovered a novel gene, which when mutated, is responsible for familial British dementia (FBD), a rare inherited disease that causes severe movement disorders and progressive dementia similar to that seen in AD. In a biochemical analysis of the amyloid fibrils found in the FBD plaques, a unique peptide named ABri was found (Vidal *et al*., 1999). A mutation at a particular point along this gene results in the production of a longer-than-normal Bri protein. The ABri peptide, which is snipped from the mutated end of the Bri protein is deposited as amyloid fibrils. These plaques are thought to lead to the neuronal dysfunction and dementia that characterizes FBD.

### Immunization with Aβ

The immune system will normally take part in the clearing of foreign protein and proteinaceous particles in the organism but the deposits associated with the above-mentioned diseases consist mainly of self-proteins, thereby rendering the role of the immune system in the control of these diseases less obvious. Further, the deposits are located in a compartment (the CNS) normally separated from the immune system, both facts suggesting that any vaccine or immunotherapeutical approach would be unsuccessful.

Nevertheless, scientists have recently attempted immunizing mice with a vaccine composed of heterologous human Aβ and a substance known to excite the immune system (Schenk et al., 1999 and WO 99/27944). The vaccine was tested in a partial transgenic mouse model of AD with a human mutated gene for APP inserted into the DNA of the mouse. The mice produced the modified APP protein and developed amyloid plaques as they grew older. This mouse model was used to test whether vaccination against the modified transgenic *human* APP had an effect on plaque build-up. In a first experiment, one group of transgenic mice was given monthly injections of the vaccine starting at 6 weeks of age and ending at 11 months. A second group of transgenic mice received no injections and served as a control group. By 13 months of age, the mice in the control group had plaques covering 2 to 6 percent of their brains. In contrast, the immunized mice had virtually no plaques.

In a second experiment, the researchers began the injections at 11 months, when some plaques had already developed. Over a 7-month period, the control transgenic mice had a 17-fold increase in the amount of plaque in their brains, whereas those who received the vaccine had a 99-percent decrease compared to the 18-month-old control transgenic mice. In some mice, some of the pre-existing plaque deposits appeared to have been removed by the treatment. It was also found that other plaque-associated damage, such as inflammation and abnormal nerve cell processes, lessened as a result of the immunization.

The above is thus a preliminary study in mice and for example, scientists need to find out whether vaccinated mice remain healthy in other respects and whether memory of those vaccinated remains normal. Furthermore, because the mouse model is not a complete representation of AD (the animals do not develop neurofibrillary tangles nor do many of their neurons die), additional studies will be necessary to determine whether humans have a similar or different reaction from mice. Another issue to consider is that the method may perhaps "cure" amyloid deposition but fail to stop development of dementia.

Technical issues present major challenges as well. For example it is unlikely that it is even possible, using this technology, to create a vaccine which enables humans to raise antibodies against their own proteins. So numerous issues of safety and effectiveness will need to be resolved before any tests in humans can be considered.

The work by Schenk *et al*. thus shows that if it was possible to generate a strong immune response towards self-proteins in proteinaceous deposits in the central nervus system such as the plaques formed in AD, it is possible to both prevent the formation of the deposits and possibly also clear already formed plaques.

## OBJECT OF THE INVENTION

The object of the present invention is to provide novel therapies against conditions characterized by deposition of amyloid, such as AD. A further object is to develop an autovaccine against amyloid, in order to obtain a novel treatment for AD and for other pathological disorders involving amyloid deposition.

## SUMMARY OF THE INVENTION

Described herein is the use of an autovaccination technology for generating strong immune responses against otherwise non-immunogenic self-proteins included in pathology-related amyloid deposits. Thereby, a strong immune response is generated against either the amyloid, against one or more of the components included in the deposits, or against one or more of the proteins responsible for amyloid formation. Described is also the preparation of such vaccines for the prevention, possible cure or alleviation of the symptoms of such diseases associated with amyloid deposits.

Thus, in its broadest and most general scope, the present invention relates to use of an agent selected from

a) at least one analogue of an animal's autologous Aβ or APP polypeptide wherein is introduced at least one isolated foreign T helper epitope ($T_H$ epitope) by means of amino acid insertion, addition, deletion, or substitution, wherein the foreign $T_H$ epitope is free from D-amino acids and is introduced into the autologous Aβ or APP as schematically shown for the P2 and P30 epitopes in Fig. 1,

b) a nucleic add sequence encoding at least one analogue defined in a), and

c) a non-pathogenic microorganism or virus which carries the nucleic acid fragment defined in b),

for the preparation of a medicament containing the agent for treating and/or preventing and/or ameliorating Alzheimer's disease or other diseases and conditions characterized by Aβ deposits in said animal.

Hence, encompassed by the present invention is the use of analogues of naturally occurring antigens, the analogues being capable of inducing cross-reactive immune responses.

The invention also relates to analogues of the Aβ and APP polypeptides as well as to nucleic acid fragments encoding a subset of these. Also immunogenic compositions comprising the analogues or the nucleic acid fragments are part of the invention.

LEGEND TO THE FIGURE

Fig. 1: Schematic depiction of Autovac variants derived from the amyloid precursor protein with the purpose of generating antibody responses against the Aβ protein Aβ-43 (or C-100). The APP is shown schematically at the top of the figure and the remaining schematic constructs show that the model epitopes P2 and P30 are substituted or inserted into various truncations of APP. In the figure, the black pattern indicates the APP signal sequence, two-way cross-hatching is the extracellular part of APP, dark vertical hatching is the transmembrane domain of APP, light vertical hatching is the intracellular domain of APP, coarse cross-hatching indicates the P30 epitope, and fine cross-hatching indicates the P2 epitope. The full line box indicates Aβ-42/43 and the full-line box and the dotted line box together indicate C-100. "Abeta" denotes Aβ.

DETAILED DISCLOSURE OF THE INVENTION

Definitions

In the following a number of terms used in the present specification and claims will be defined and explained in detail in order to clarify the metes and bounds of the invention.

The terms "amyloid" and "amyloid protein" which are used interchangeably herein denote a class of proteinaceous unbranched fibrils of indeterminate length. Amyloid fibrils display characteristic staining with Congo Red and share a cross-β structure in which the polypeptide chain is organized in β-sheets. Amyloid is generally derived from amyloidogenic proteins which have very different precursor structures but which can all undergo a structural conversion to a misfolded form that is the building block of the β-sheet helix protofilament. Normally, the diameter of amyloid fibrils varies between about 70 to about 120 Å.

The term "amyloidogenic protein" is intended to denote a polypeptide which is involved in the formation of amyloid deposits, either by being part of the deposits as such or by being part of the biosynthetic pathway leading to the formation of the deposits. Hence, examples of amyloidogenic proteins are APP and Aβ, but also proteins involved in the metabolism of these may be amyloidogenic proteins. A number of amyloidogenic polypeptides are discussed in detail herein.

An "amyloid polypeptide" is herein intended to denote polypeptides comprising the amino acid sequence of the above-discussed amyloidogenic proteins derived from humans or other mammals (or truncates thereof sharing a substantial amount of B-cell epitopes with an intact amyloidogenic protein) - an amyloidogenic polypeptide can therefore e.g. comprise substantial parts of a precursor for the amyloidogenic polypeptide (in the case of Aβ, one possible amyloid polypeptide could be APP derived). Also unglycosylated forms of amyloidogenic polypeptides which are prepared in prokaryotic system are included within the boundaries of the term as are forms having varying glycosylation patterns due to the use of e.g. yeasts or other non-mammalian eukaryotic expression systems. It should, however, be noted that when using the term "an amyloidogenic polypeptide" it is intended that the polypeptide in question is normally non-immunogenic when presented to the animal to be treated. In other words, the amyloidogenic polypeptide is a self-protein or is an analogue of such a self-protein which will not normally give rise to an immune response against the amyloidogenic of the animal in question.

An "analogue of an amyloidogenic polypeptide" is an amyloidogenic polypeptide, which has been subjected to changes in its primary structure. Such a change can be in the form of insertions and/or deletions and/or substitutions in the amyloidogenic polypeptide's amino acid sequence. Also encompassed by the term are derivatized amyloidogenic molecules, cf. the discussion below of modifications of amyloidogenic polypeptides. In case the amyloidogenic polypeptide is an amyloid or a precursor therefore, the analogue may be constructed so as to be less able or even unable to elicit antibodies against the normal precursor protein(s) of the amyloid, thereby avoiding undesired interference with the (physiologically normal) non-aggregated form of the polypeptide being a precursor of the amyloid protein.

It should be noted that the use as a vaccine in a human of a xeno-analogue (e.g. a canine or porcine analogue) of a human amyloidogenic polypeptide can be imagined to produce the desired immunity against the amyloidogenic polypeptide. Such use of an xeno-analogue for immunization is also considered part of the invention.

The term "polypeptide" is in the present context intended to mean both short peptides of from 2 to 10 amino acid residues, oligopeptides of from 11 to 100 amino acid residues, and polypeptides of more than 100 amino acid residues. Furthermore, the term is also intended to include proteins, *i.e.* functional biomolecules comprising at least one polypeptide; when comprising at least two polypeptides, these may form complexes, be covalently linked, or may be non-covalently linked. The polypeptide(s) in a protein can be glycosylated and/or lipidated and/or comprise prosthetic groups.

The terms "T-lymphocyte" and "T-cell" will be used interchangeably for lymphocytes of thymic origin which are responsible for various cell mediated immune responses as well as for helper activity in the humoral immune response. Likewise, the terms "B-lymphocyte" and "B-cell" will be used interchangeably for antibody-producing lymphocytes.

The term "subsequence" means any consecutive stretch of at least 3 amino acids or, when relevant, of at least 3 nucleotides, derived directly from a naturally occurring amyloid amino acid sequence or nucleic acid sequence, respectively.

The term "animal" is in the present context in general intended to denote an animal species (preferably mammalian), such as *Homo sapiens, Canis domesticus,* etc. and not just one single animal. However, the term also denotes a population of such an animal species, since it is important that the individuals immunized according to the method of the invention all harbour substantially the same amyloidogenic polypeptide allowing for immunization of the animals with the same immunogen(s). If, for instance, genetic variants of the amyloidogenic polypeptide exists in different human population it may be necessary to use different immunogens in these different populations in order to be able to break the autotolerance towards the amyloidogenic polypeptide in each population in an optimum fashion. It will be clear to the skilled person that an animal in the present context is a living being which has an immune system. It is preferred that the animal is a vertebrate, such as a mammal.

By the term *"in vivo* down-regulation of amyloid" is herein meant reduction in the living organism of the total amount of deposited amyloid of the relevant type. The down-regulation can be obtained by means of several mechanisms: Of these, simple interference with amyloid by antibody binding so as to prevent misaggregation is the most simple. However, it is also within the scope of the present invention that the antibody binding results in removal of amyloid by scavenger cells (such as macrophages and other phagocytic cells) and that the antibodies interfer with other amyloidogenic polypeptides which lead to amyloid formation.

The expression "effecting presentation ... to the immune system" is intended to denote that the animal's immune system is subjected to an immunogenic challenge in a controlled manner. As will appear from the disclosure below, such challenge of the immune system can be effected in a number of ways of which the most important are vaccination with polypeptide containing "pharmaccines" (i.e. a vaccine which is administered to treat or ameliorate ongoing disease) or nucleic acid "pharmaccine" vaccination. The important result to achieve is that immune competent cells in the animal are confronted with the antigen in an immunologically effective manner, whereas the precise mode of achieving this result is of less importance to the inventive idea underlying the present invention.

The term "immunogenically effective amount" has its usual meaning in the art, i.e. an amount of an immunogen, which is capable of inducing an immune response that significantly engages pathogenic agents sharing immunological features with the immunogen.

When using the expression that the amyloidogenic polypeptide has been "modified" is herein meant a chemical modification of the polypeptide, which constitutes the backbone of the amyloidogenic polypeptide. Such a modification can e.g. be derivatization (e.g. alkylation) of certain amino acid residues in the sequence of the amyloidogenic polypeptide, but as will be appreciated from the disclosure below, the preferred modifications comprise changes of the primary structure of the amino acid sequence.

When discussing "autotolerance towards an amyloidogenic polypeptide" it is understood that since the amyloidogenic polypeptide is a self-protein in the population to be vaccinated, normal individuals in the population do not mount an immune response against the amyloidogenic polypeptide; it cannot be excluded, though, that occasional individuals in an animal population might be able to produce antibodies against native amyloidogenic polypeptide, e.g. as part of an autoimmune disorder. At any rate, an animal will normally only be autotolerant towards its own amyloidogenic polypeptide, but it cannot be excluded that analogues derived from other animal species or from a population having a different phenotype would also be tolerated by said animal.

A "foreign T-cell epitope" (or: "foreign T-lymphocyte epitope") is a peptide which is able to bind to an MHC molecule and which stimulates T-cells in an animal species. Preferred foreign T-cell epitopes in the invention are "promiscuous" epitopes, i.e. epitopes which bind to a substantial fraction of a particular class of MHC molecules in an animal species or population. Only a very limited number of such promiscuous T-cell epitopes are known, and they will be discussed in detail below. Promiscuous T-cell epitopes are also denoted "universal" T-cell epitopes. It should be noted that in order for the immunogens which are used according to the present invention to be effective in as large a fraction of an animal population as possible, it may be necessary to 1) insert several foreign T-cell epitopes in the same analogue or 2) prepare several analogues wherein each analogue has a different promiscuous epitope inserted. It should be noted also that the concept of foreign T-cell epitopes also encompasses use of cryptic T-cell epitopes, i.e. epitopes which are derived from a self-protein and which only exerts immunogenic behaviour when existing in isolated form without being part of the self-protein in question.

A "foreign T helper lymphocyte epitope" (a foreign $T_H$ epitope) is a foreign T cell epitope, which binds an MHC Class II molecule and can be presented on the surface of an antigen presenting cell (APC) bound to the MHC Class II molecule.

A "functional part" of a (bio)molecule is in the present context intended to mean the part of the molecule which is responsible for at least one of the biochemical or physiological effects exerted by the molecule. It is well-known in the art that many enzymes and other effector molecules have an active site which is responsible for the effects exerted by the molecule in question. Other parts of the molecule may serve a stabilizing or solubility enhancing purpose and can therefore be left out if these purposes are not of relevance in the context of a certain embodiment of the present invention. For instance it is possible to use certain cytokines as a modifying moiety in an amyloidogenic polypeptide (cf. the detailed discussion below), and in such a case, the issue of stability may be irrelevant since the coupling to the amyloidogenic polypeptide may provide the stability necessary.

The term "adjuvant" has its usual meaning in the art of vaccine technology, i.e. a substance or a composition of matter which is 1) not in itself capable of mounting a specific immune response against the immunogen of the vaccine, but which is 2) nevertheless capable of enhancing the immune response against the immunogen. Or, in other words, vaccination with the adjuvant alone does not provide an immune response against the immunogen, vaccination with the immunogen may or may not give rise to an immune response against the immunogen, but the combined vaccination with immunogen and adjuvant induces an immune response against the immunogen which is stronger than that induced by the immunogen alone.

"Targeting" of a molecule is in the present context intended to denote the situation where a molecule upon introduction in the animal will appear preferentially in certain tissue(s) or will be preferentially associated with certain cells or cell types. The effect can be accomplished in a number of ways including formulation of the molecule in composition facilitating targeting or by introduction in the molecule of groups, which facilitate targeting. These issues will be discussed in detail below.

"Stimulation of the immune system" means that a substance or composition of matter exhibits a general, non-specific immunostimulatory effect. A number of adjuvants and putative adjuvants (such as certain cytokines) share the ability to stimulate the immune system. The result of using an immunostimulating agent is an increased "alertness" of the immune system meaning that simultaneous or subsequent immunization with an immunogen induces a significantly more effective immune response compared to isolated use of the immunogen

<u>Preferred embodiments of amyloid down-regulation</u>

It is preferred that the amyloidogenic polypeptide used as an immunogen in the method of the invention is a modified molecule wherein at least one change is present in the amino acid sequence of the amyloidogenic polypeptide, since the chances of obtaining the all-important breaking of autotolerance towards the amyloidogenic polypeptide is greatly facilitated that way - this is e.g. evident from the results presented in Example 2 herein, where immunization with wild-type Aβ is compared to immunization with an Aβ variant molecule. It should be noted that the use of a modified molecule does not exclude the possibility of using such a modified amyloidogenic polypeptide in formulations which further facilitate the breaking of autotolerance against the amyloidogenic polypeptide, e.g. formulations containing adjuvants.

It has been shown (in Dalum I *et al.*, 1996, J. Immunol. 157: 4796-4804) that potentially self-reactive B-lymphocytes recognizing self-proteins are physiologically present in normal individuals. However, in order for these B-lymphocytes to be induced to actually produce antibodies reactive with the relevant self-proteins, assistance is needed from cytokine producing T-helper lymphocytes ($T_H$-cells or $T_H$-lymphocytes). Normally this help is not provided because T-lymphocytes in general do not recognize T-cell epitopes derived from self-proteins when presented by antigen presenting cells (APCs). However, by providing an element of "foreignness" in a self-protein (i.e. by introducing an immunologically significant modification), T-cells recognizing the foreign element are activated upon recognizing the foreign epitope on an APC (such as, initially, a mononuclear cell). Polyclonal B-lymphocytes (which are also APCs) capable of recognising self-epitopes on the modified self-protein also internalise the antigen and subsequently presents the foreign T-cell epitope(s) thereof, and the activated T-lymphocytes subsequently provide cytokine help to these self-reactive polyclonal B-lymphocytes. Since the antibodies produced by these polyclonal B-lymphocytes are reactive with different epitopes on the modified polypeptide, including those which are also present in the native polypeptide, an antibody cross-reactive with the non-modified self-protein is induced. In conclusion, the T-lymphocytes can be led to act as if the population of polyclonal B-lymphocytes have recognised an entirely foreign antigen, whereas in fact only the inserted epitope(s) is/are foreign to the host. In this way, antibodies capable of *cross-reacting* with non-modified self-antigens are induced.

Several ways of modifying a peptide self-antigen in order to obtain breaking of autotolerance are known in the art. Hence, according to the invention, the modification can include that

- at least one first moiety is introduced which effects targeting of the modified molecule to an antigen presenting cell (APC), and/or

- at least one second moiety is introduced which stimulates the immune system, and/or
- at least one third moiety is introduced which optimizes presentation of the modified amyloidogenic polypeptide to the immune system.

However, all these modifications should be carried out while maintaining a substantial fraction of the original B-lymphocyte epitopes in the amyloidogenic polypeptide, since the B-lymphocyte recognition of the native molecule is thereby enhanced.

In one preferred embodiment, side groups (in the form of foreign T-cell epitopes or the above-mentioned first, second and third moieties) are covalently or non-covalently introduced. This is to mean that stretches of amino acid residues derived from the amyloidogenic polypeptide are derivatized without altering the primary amino acid sequence, or at least without introducing changes in the peptide bonds between the individual amino acids in the chain.

An alternative, and preferred, embodiment utilises amino acid substitution and/or deletion and/or insertion and/or addition (which may be effected by recombinant means or by means of peptide synthesis). One especially preferred version of this embodiment is the technique described in WO 95/05849, which discloses a method for down-regulating self-proteins by immunising with analogues of the self-proteins wherein a number of amino acid sequence (s) has been substituted with a corresponding number of amino acid sequence(s) which each comprise a foreign immunodominant T-cell epitope, while at the same time maintaining the overall tertiary structure of the self-protein in the analogue. For the purposes of the present invention, it is however sufficient if the modification (be it an insertion, addition, deletion or substitution) gives rise to a foreign T-cell epitope and at the same time preserves a substantial number of the B-cell epitopes in the amyloidogenic polypeptide. However, in order to obtain maximum efficacy of the immune response induced, it is preferred that the overall tertiary structure of the amyloidogenic polypeptide is maintained in the modified molecule.

Thus, included in the invention are modified amyloidogenic polypeptides obtained by omission of parts of the sequence of the amyloidogenic polypeptide which e.g. exhibit adverse effects *in vivo* or omission of parts which are normally intracellular and thus could give rise to undesired immunological reactions.

One preferred version of the constructs outlined above are, when applicable, those where the B-cell epitope containing subsequence of an amyloid protein is not extracellularly exposed in the precursor polypeptide from which the amyloid is derived. By making such a choice of the amyloid epitopes, it is ensured that antibodies are not generated which would be reactive with the cells producing the amyloid precursor and thereby the immune response which is generated becomes limited to an immune response against the undesired amyloid deposits. A similar choice can, when applicable, be made for other amyloidogenic polypeptides than amyloid. In these cases it will e.g. be feasible to induce immunity against epitopes of the amyloidogenic polypeptide which are only exposed to the extracellular phase when being free from any coupling to the cells from which they are produced.

Maintenance of a substantial fraction of B-cell epitopes or even the overall tertiary structure of a protein which is subjected to modification as described herein can be achieved in several ways. One is simply to prepare a polyclonal antiserum directed against the amyloidogenic polypeptide (e.g. an antiserum prepared in a rabbit) and thereafter use this antiserum as a test reagent (e.g. in a competitive ELISA) against the modified proteins which are produced. Modified versions (analogues) which react to the same extent with the antiserum as does the amyloidogenic polypeptide must be regarded as having the same overall tertiary structure as the amyloidogenic polypeptide whereas analogues exhibiting a limited (but still significant and specific) reactivity with such an antiserum are regarded as having maintained a substantial fraction of the original B-cell epitopes.

Alternatively, a selection of monoclonal antibodies reactive with distinct epitopes on the amyloidogenic polypeptide can be prepared and used as a test panel. This approach has the advantage of allowing 1) an epitope mapping of the amyloidogenic polypeptide and 2) a mapping of the epitopes which are maintained in the analogues prepared.

Of course, a third approach would be to resolve the 3-dimensional structure of the amyloidogenic polypeptide or of a biologically active truncate thereof (cf. above) and compare this to the resolved three-dimensional structure of the analogues prepared. Three-dimensional structure can be resolved by the aid of X-ray diffraction studies and NMR-spectroscopy. Further information relating to the tertiary structure can to some extent be obtained from circular dichroism studies which have the advantage of merely requiring the polypeptide in pure form (whereas X-ray diffraction requires the provision of crystallized polypeptide and NMR requires the provision of isotopic variants of the polypeptide) in order to provide useful information about the tertiary structure of a given molecule. However, ultimately X-ray diffraction and/or NMR are necessary to obtain conclusive data since circular dichroism can only provide indirect evidence of correct 3-dimensional structure via information of secondary structure elements.

One preferred embodiment of the invention utilises multiple presentations of B-lymphocyte epitopes of the amyloidogenic polypeptide (i.e. formula I wherein at least one B-cell epitope is present in two positions). This effect can be achieved in various ways, e.g. by simply preparing fusion polypeptides comprising the structure (amyloidogenic

polypeptide)$_m$, where m is an integer ≥ 2 and then introduce the modifications discussed herein in at least one of the amyloid sequences. It is preferred that the modifications introduced includes at least one duplication of a B-lymphocyte epitope and/or the introduction of a hapten. These embodiments including multiple presentations of selected epitopes are especially preferred in situations where merely minor parts of the amyloidogenic polypeptide are useful as constituents in a vaccine agent.

As mentioned above, the introduction of a foreign T-cell epitope can be accomplished by introduction of at least one amino acid insertion, addition, deletion, or substitution. Of course, the normal situation will be the introduction of more than one change in the amino acid sequence (e.g. insertion of or substitution by a complete T-cell epitope) but the important goal to reach is that the analogue, when processed by an antigen presenting cell (APC), will give rise to such a foreign immunodominant T-cell epitope being presented in context of an MCH Class II molecule on the surface of the APC. Thus, if the amino acid sequence of the amyloidogenic polypeptide in appropriate positions comprises a number of amino acid residues which can also be found in a foreign $T_H$ epitope then the introduction of a foreign $T_H$ epitope can be accomplished by providing the remaining amino acids of the foreign epitope by means of amino acid insertion, addition, deletion and substitution. In other words, it is not necessary to introduce a complete $T_H$ epitope by insertion or substitution in order to fulfill the purpose of the present invention.

It is preferred that the number of amino acid insertions, deletions, substitutions or additions is at least 2, such as 3, 4, 5, 6, 7, 8, 9, 10, 11, 12, 13, 14, 15, 16, 17, 18, 19, 20, and 25 insertions, substitutions, additions or deletions. It is furthermore preferred that the number of amino acid insertions, substitutions, additions or deletions is not in excess of 150, such as at most 100, at most 90, at most 80, and at most 70. It is especially preferred that the number of substitutions, insertions, deletions, or additions does not exceed 60, and in particular the number should not exceed 50 or even 40. Most preferred is a number of not more than 30. With respect to amino acid additions, it should be noted that these, when the resulting construct is in the form of a fusion polypeptide, is often considerably higher than 150.

Preferred embodiments of the invention includes modification by introducing at least one foreign immunodominant T-cell epitope. It will be understood that the question of immune dominance of a T-cell epitope depends on the animal species in question. As used herein, the term "immunodominance" simply refers to epitopes which in the vaccinated individual/population gives rise to a significant immune response, but it is a well-known fact that a T-cell epitope which is immunodominant in one individual/population is not necessarily immunodominant in another individual of the same species, even though it may be capable of binding MHC-II molecules in the latter individual. Hence, for the purposes of the present invention, an immune dominant T-cell epitope is a T-cell epitope which will be effective in providing T-cell help when present in an antigen. Typically, immune dominant T-cell epitopes has as an inherent feature that they will substantially always be presented bound to an MHC Class II molecule, irrespective of the polypeptide wherein they appear.

Another important point is the issue of MHC restriction of T-cell epitopes. In general, naturally occurring T-cell epitopes are MHC restricted, i.e. a certain peptides constituting a T-cell epitope will only bind effectively to a subset of MHC Class II molecules. This in turn has the effect that in most cases the use of one specific T-cell epitope will result in a vaccine component which is only effective in a fraction of the population, and depending on the size of that fraction, it can be necessary to include more T-cell epitopes in the same molecule, or alternatively prepare a multicomponent vaccine wherein the components are variants of the amyloidogenic polypeptide which are distinguished from each other by the nature of the T-cell epitope introduced.

If the MHC restriction of the T-cells used is completely unknown (for instance in a situation where the vaccinated animal has a poorly defined MHC composition), the fraction of the population covered by a specific vaccine composition can be determined by means of the following formula

$$f_{population} = 1 - \prod_{i=1}^{n}(1 - p_i) \qquad \text{(II)}$$

-where $p_i$ is the frequency in the population of responders to the $i^{th}$ foreign T-cell epitope present in the vaccine composition, and n is the total number of foreign T-cell epitopes in the vaccine composition. Thus, a vaccine composition containing 3 foreign T-cell epitopes having response frequencies in the population of 0.8, 0.7, and 0.6, respectively, would give

$$1 - 0.2 \times 0.3 \times 0.4 = 0.976$$

-*i.e.* 97.6 percent of the population will statistically mount an MHC-II mediated response to the vaccine.

The above formula does not apply in situations where a more or less precise MHC restriction pattern of the peptides used is known. If, for instance a certain peptide only binds the human MHC-II molecules encoded by HLA-DR alleles DR1, DR3, DR5, and DR7, then the use of this peptide together with another peptide which binds the remaining MHC-II molecules encoded by HLA-DR alleles will accomplish 100% coverage in the population in question. Likewise, if the second peptide only binds DR3 and DR5, the addition of this peptide will not increase the coverage at all. If one bases the calculation of population response purely on MHC restriction of T-cell epitopes in the vaccine, the fraction of the population covered by a specific vaccine composition can be determined by means of the following formula:

$$f_{population} = 1 - \prod_{j=1}^{3} (1 - \varphi_j)^2 \qquad \text{(III)}$$

-wherein $\varphi_j$ is the sum of frequencies in the population of allelic haplotypes encoding MHC molecules which bind any one of the T-cell epitopes in the vaccine and which belong to the $j^{th}$ of the 3 known HLA loci (DP, DR and DQ); in practice, it is first determined which MHC molecules will recognize each T-cell epitope in the vaccine and thereafter these are listed by type (DP, DR and DQ) - then, the individual frequencies of the different listed allelic haplotypes are summed for each type, thereby yielding $\varphi_1$, $\varphi_2$, and $\varphi_3$.

It may occur that the value $p_i$ in formula II exceeds the corresponding theoretical value $p_i$:

$$\pi_i = 1 - \prod_{j=1}^{3} (1 - \nu_j)^2 \qquad \text{(IV)}$$

-wherein $U_j$ is the sum of frequencies in the population of allelic haplotype encoding MHC molecules which bind the $i^{th}$ T-cell epitope in the vaccine and which belong to the $j^{th}$ of the 3 known HLA loci (DP, DR and DQ). This means that in 1-$p_i$ of the population is a frequency of responders of $f_{residual_i} = (p_i - p_i)/(1 - p_i)$. Therefore, formula III can be adjusted so as to yield formula V:

$$f_{population} = 1 - \prod_{j=1}^{3} (1 - \varphi_j)^2 + \left(1 - \prod_{i=1}^{n} (1 - f_{residual_i})\right) \qquad \text{(V)}$$

-where the term 1-$\varphi_{residual\text{-}i}$ is set to zero if negative. It should be noted that formula V requires that all epitopes have been haplotype mapped against identical sets of haplotypes.

Therefore, when selecting T-cell epitopes to be introduced in the analogue, it is important to include all knowledge of the epitopes which is available: 1) The frequency of responders in the population to each epitope, 2) MHC restriction data, and 3) frequency in the population of the relevant haplotypes.

There exist a number of naturally occurring "promiscuous" T-cell epitopes which are active in a large proportion of individuals of an animal species or an animal population and these are preferably introduced in the vaccine thereby reducing the need for a very large number of different analogues in the same vaccine.

The promiscuous epitope can according to the invention be a naturally occurring human T-cell epitope such as epitopes from tetanus toxoid (e.g. the P2 and P30 epitopes), diphtheria toxoid, Influenza virus hemagluttinin (HA), and *P. falciparum* CS antigen.

Over the years a number of other promiscuous T-cell epitopes have been identified. Especially peptides capable of binding a large proportion of HLA-DR molecules encoded by the different HLA-DR alleles have been identified and these are all possible T-cell epitopes to be introduced in the analogues used according to the present invention. Cf. also the epitopes discussed in the following references which are hereby all incorporated by reference herein: WO 98/23635 (Frazer IH et *al*., assigned to The University of Queensland); Southwood S *et. al*, 1998, J. Immunol. **160:** 3363-3373; Sinigaglia F *et al*., 1988, Nature **336:** 778-780; Chicz RM *et al*., 1993, J. Exp. Med **178:** 27-47; Hammer J *et al*., 1993, Cell **74:** 197-203; and Falk K et *al*., 1994, Immunogenetics **39:** 230-242. The latter reference also deals with HLA-DQ and -DP ligands. All epitopes listed in these 5 references are relevant as candidate natural epitopes to be used in the present invention, as are epitopes which share common motifs with these.

Alternatively, the epitope can be any artificial T-cell epitope which is capable of binding a large proportion of MHC Class II molecules. In this context the pan DR epitope peptides ("PADRE") described in WO 95/07707 and in the corresponding paper Alexander J *et al.*, 1994, Immunity 1: 751-761 (both disclosures are incorporated by reference herein) are interesting candidates for epitopes to be used according to the present invention. It should be noted that the most effective PADRE peptides disclosed in these papers carry D-amino acids in the C- and N-termini in order to improve stability when administered. However, the present invention primarily aims at incorporating the relevant epitopes as part of the modified amyloidogenic polypeptide which should then subsequently be broken down enzymatically inside the lysosomal compartment of APCs to allow subsequent presentation in the context of an MHC-II molecule and therefore it is not expedient to incorporate D-amino acids in the epitopes used in the present invention.

One especially preferred PADRE peptide is the one having the amino acid sequence AKFVAAWTLKAAA or an immunologically effective subsequence thereof. This, and other epitopes having the same lack of MHC restriction are preferred T-cell epitopes which should be present in the analogues used in the inventive method. Such super-promiscuous epitopes will allow for the most simple embodiments of the invention wherein only one single modified amyloidogenic polypeptide is presented to the vaccinated animal's immune system.

As mentioned above, the modification of the amyloidogenic polypeptide can also include the introduction of a first moiety which targets the modified amyloidogenic polypeptide to an APC or a B-lymphocyte. For instance, the first moiety can be a specific binding partner for a B-lymphocyte specific surface antigen or for an APC specific surface antigen. Many such specific surface antigens are known in the art. For instance, the moiety can be a carbohydrate for which there is a receptor on the B-lymphocyte or the APC (e.g. mannan or mannose). Alternatively, the second moiety can be a hapten. Also an antibody fragment which specifically recognizes a surface molecule on APCs or lymphocytes can be used as a first moiety (the surface molecule can e.g. be an FCγ receptor of macrophages and monocytes, such as FCγRI or, alternatively any other specific surface marker such as CD40 or CTLA-4). It should be noted that all these exemplary targeting molecules can be used as part of an adjuvant also, cf. below.

As an alternative or supplement to targeting the modified amyloidogenic polypeptide to a certain cell type in order to achieve an enhanced immune response, it is possible to increase the level of responsiveness of the immune system by including the above-mentioned second moiety which stimulates the immune system. Typical examples of such second moieties are cytokines, and heat-shock proteins or molecular chaperones, as well as effective parts thereof.

Suitable cytokines to be used according to the invention are those which will normally also function as adjuvants in a vaccine composition, *i.e.* for instance interferon γ (IFN-γ), interleukin 1 (IL-1), interleukin 2 (IL-2), interleukin 4 (IL-4), interleukin 6 (IL-6), interleukin 12 (IL-12), interleukin 13 (IL-13), interleukin 15 (IL-15), and granulocyte-macrophage colony stimulating factor (GM-CSF); alternatively, the functional part of the cytokine molecule may suffice as the second moiety. With respect to the use of such cytokines as adjuvant substances, cf. the discussion below.

According to the invention, suitable heat-shock proteins or molecular chaperones used as the second moiety can be HSP70, HSP90, HSC70, GRP94 (also known as gp96, cf. Wearsch PA *et al.* 1998, Biochemistry **37:** 5709-19), and CRT (calreticulin).

Alternatively, the second moiety can be a toxin, such as listeriolycin (LLO), lipid A and heat-labile enterotoxin. Also, a number of mycobacterial derivatives such as MDP (muramyl dipeptide), CFA (complete Freund's adjuvant) and the trehalose diesters TDM and TDE are interesting possibilities.

Also the possibility of introducing a third moiety which enhances the presentation of the modified amyloidogenic polypeptide to the immune system is an important embodiment of the invention. The art has shown several examples of this principle. For instance, it is known that the palmitoyl lipidation anchor in the *Borrelia burgdorferi* protein OspA can be utilised so as to provide self-adjuvating polypeptides (cf. e.g. WO 96/40718) - it seems that the lipidated proteins form up micelle-like structures with a core consisting of the lipidation anchor parts of the polypeptides and the remaining parts of the molecule protruding therefrom, resulting in multiple presentations of the antigenic determinants. Hence, the use of this and related approaches using different lipidation anchors (e.g. a myristyl group, a myristyl group, a farnesyl group, a geranyl-geranyl group, a GPI-anchor, and an N-acyl diglyceride group) are preferred embodiments of the invention, especially since the provision of such a lipidation anchor in a recombinantly produced protein is fairly straightforward and merely requires use of e.g. a naturally occurring signal sequence as a fusion partner for the modified amyloidogenic polypeptide. Another possibility is use of the C3d fragment of complement factor C3 or C3 itself (cf. Dempsey *et al*., 1996, Science **271,** 348-350 and Lou & Kohler, 1998, Nature Biotechnology **16,** 458-462).

An alternative embodiment of the invention which also results in the preferred presentation of multiple (e.g. at least 2) copies of the important epitopic regions of the amyloidogenic polypeptide to the immune system is the covalent coupling of the amyloidogenic polypeptide, subsequence or variants thereof to certain molecules. For instance, polymers can be used, e.g. carbohydrates such as dextran, cf. e.g. Lees A *et al*., 1994, Vaccine **12:** 1160-1166; Lees A *et al*., 1990, J Immunol. **145:** 3594-3600, but also mannose and mannan are useful alternative. Integral membrane proteins from e.g. *E. coli* and other bacteria are also useful conjugation partners. The traditional carrier molecules such as keyhole limpet hemocyanin (KLH), tetanus toxoid, diphtheria toxoid, and bovine serum albumin (BSA) are also preferred and useful conjugation partners.

Considerations underlying chosen areas of introducing modifications in amyloidogenic polypeptides are a) preservation of known and predicted B-cell epitopes, b) preservation of tertiary structure, c) avoidance of B-cell epitopes present on "producer cells" etc. At any rate, as discussed above, it is fairly easy to screen a set of modified amyloidogenic molecules which have all been subjected to introduction of a T-cell epitope in different locations.
Since the present invention involves down-regulation of human Aβ, it is consequently preferred that the amyloid polypeptide discussed above is a human Aβ polypeptide. In this embodiment, it is especially preferred that the human amyloidogenic polypeptide has been modified by substituting at least one amino acid sequence in SEQ ID NO: 2 with at least one amino acid sequence of equal or different length and containing a foreign $T_H$ epitope. Examples of modified amyloidogenic APP and Aβ are shown schematically in Fig. 1 using the P2 and P30 epitopes as examples. The rationale behind such constructs is discussed in detail in the example.

Formulation of the modified amyloidogenic polypeptides

When effecting presentation of the amyloidogenic polypeptide or the modified amyloidogenic polypeptide to an animal's immune system by means of administration thereof to the animal, the formulation of the polypeptide follows the principles generally acknowledged in the art.
Preparation of vaccines which contain peptide sequences as active ingredients is generally well understood in the art, as exemplified by U.S. Patents 4,608,251; 4,601,903; 4,599,231; 4,599,230; 4,596,792; and 4,578,770, all incorporated herein by reference. Typically, such vaccines are prepared as injectables either as liquid solutions or suspensions; solid forms suitable for solution in, or suspension in, liquid prior to injection may also be prepared. The preparation may also be emulsified. The active immunogenic ingredient is often mixed with excipients which are pharmaceutically acceptable and compatible with the active ingredient. Suitable excipients are, for example, water, saline, dextrose, glycerol, ethanol, or the like, and combinations thereof. In addition, if desired, the vaccine may contain minor amounts of auxiliary substances such as wetting or emulsifying agents, pH buffering agents, or adjuvants which enhance the effectiveness of the vaccines; of the detailed discussion of adjuvants below.
The vaccines are conventionally administered parenterally, by injection, for example, either subcutaneously, intracutaneously, intradermally, subdermally or intramuscularly. Additional formulations which are suitable for other modes of administration include suppositories and, in some cases, oral, buccal, sublinqual, intraperitoneal, intravaginal, anal, epidural, spinal, and intracranial formulations. For suppositories, traditional binders and carriers may include, for example, polyalkalene glycols or triglycerides; such suppositories may be formed from mixtures containing the active ingredient in the range of 0.5% to 10%, preferably 1-2%. Oral formulations include such normally employed excipients as, for example, pharmaceutical grades of mannitol, lactose, starch, magnesium stearate, sodium saccharine, cellulose, magnesium carbonate, and the like. These compositions take the form of solutions, suspensions, tablets, pills, capsules, sustained release formulations or powders and contain 10-95% of active ingredient, preferably 25-70%. For oral formulations, cholera toxin is an interesting formulation partner (and also a possible conjugation partner).
The polypeptides may be formulated into the vaccine as neutral or salt forms. Pharmaceutically acceptable salts include acid addition salts (formed with the free amino groups of the peptide) and which are formed with inorganic acids such as, for example, hydrochloric or phosphoric acids, or such organic acids as acetic, oxalic, tartaric, mandelic, and the like. Salts formed with the free carboxyl groups may also be derived from inorganic bases such as, for example, sodium, potassium, ammonium, calcium, or ferric hydroxides, and such organic bases as isopropylamine, trimethylamine, 2-ethylamino ethanol, histidine, procaine, and the like.
The vaccines are administered in a manner compatible with the dosage formulation, and in such amount as will be therapeutically effective and immunogenic. The quantity to be administered depends on the subject to be treated, including, e.g., the capacity of the individual's immune system to mount an immune response, and the degree of protection desired. Suitable dosage ranges are of the order of several hundred micrograms active ingredient per vaccination with a preferred range from about 0.1 μg to 2,000 μg (even though higher amounts in the 1-10 mg range are contemplated), such as in the range from about 0.5 μg to 1,000 μg, preferably in the range from 1 μg to 500 μg and especially in the range from about 10 μg to 100 μg. Suitable regimens for initial administration and booster shots are also variable but are typified by an initial administration followed by subsequent inoculations or other administrations.
The manner of application may be varied widely. Any of the conventional methods for administration of a vaccine are applicable. These include oral application on a solid physiologically acceptable base or in a physiologically acceptable dispersion, parenterally, by injection or the like. The dosage of the vaccine will depend on the route of administration and will vary according to the age of the person to be vaccinated and the formulation of the antigen.
Some of the polypeptides of the vaccine are sufficiently immunogenic in a vaccine, but for some of the others the immune response will be enhanced if the vaccine further comprises an adjuvant substance.
Various methods of achieving adjuvant effect for the vaccine are known. General principles and methods are detailed in "The Theory and Practical Application of Adjuvants", 1995, Duncan E.S. Stewart-Tull (ed.), John Wiley & Sons Ltd, ISBN 0-471-95170-6, and also in "Vaccines: New Generationn Immunological Adjuvants", 1995, Gregoriadis G *et al*. (eds.), Plenum Press, New York, ISBN 0-306-45283-9, both of which are hereby incorporated by reference herein.

It is especially preferred to use an adjuvant which can be demonstrated to facilitate breaking of the autotolerance to autoantigens; in fact, this is essential in cases where unmodified amyloidogenic polypeptide is used as the active ingredient in the autovaccine. Non-limiting examples of suitable adjuvants are selected from the group consisting of an immune targeting adjuvant; an immune modulating adjuvant such as a toxin, a cytokine, and a mycobacterial derivative; an oil formulation; a polymer; a micelle forming adjuvant; a saponin; an immunostimulating complex matrix (ISCOM matrix); a particle; DDA; aluminium adjuvants; DNA adjuvants; γ-inulin; and an encapsulating adjuvant. In general it should be noted that the disclosures above which relate to compounds and agents useful as first, second and third moieties in the analogues also refer *mutatis mutandis* to their use in the adjuvant of a vaccine of the invention.

The application of adjuvants include use of agents such as aluminum hydroxide or phosphate (alum), commonly used as 0.05 to 0.1 percent solution in buffered saline, admixture with synthetic polymers of sugars (e.g. Carbopol®) used as 0.25 percent solution, aggregation of the protein in the vaccine by heat treatment with temperatures ranging between 70° to 101°C for 30 second to 2 minute periods respectively and also aggregation by means of cross-linking agents are possible. Aggregation by reactivation with pepsin treated antibodies (Fab fragments) to albumin, mixture with bacterial cells such as C. *parvum* or endotoxins or lipopolysaccharide components of gram-negative bacteria, emulsion in physiologically acceptable oil vehicles such as mannide mono-bleate (Aracel A) or emulsion with 20 percent solution of a perfluorocarbon (Fluosol-DA) used as a block substitute may also be employed. Admixture with oils such as squalene and IFA is also preferred.

According to the invention DDA (dimethyldioctadecylammonium bromide) is an interesting candidate for an adjuvant as is DNA and γ-inulin, but also Freund's complete and incomplete adjuvants as well as *quillaja* saponins such as QuilA and QS21 are interesting as is RIBI. Further possibilities are monophosphoryl lipid A (MPL), the above mentioned C3 and C3d, and muramyl dipeptide (MDP).

Liposome formulations are also known to confer adjuvant effects, and therefore liposome adjuvants are preferred according to the invention.

Also immunostimulating complex matrix type (ISCOM® matrix) adjuvants are preferred choices according to the invention, especially since it has been shown that this type of adjuvants are capable of up-regulating MHC Class II expression by APCs. An ISCOM® matrix consists of (optionally fractionated) saponins (triterpenoids) from *Quillaja saponaria,* cholesterol, and phospholipid. When admixed with the immunogenic protein, the resulting particulate formulation is what is known as an ISCOM particle where the saponin constitutes 60-70% w/w, the cholesterol and phospholipid 10-15% w/w, and the protein 10-15% w/w. Details relating to composition and use of immunostimulating complexes can e.g. be found in the above-mentioned text-books dealing with adjuvants, but also Morein B *et al*., 1995, Clin. Immunother. **3:** 461-475 as well as Barr IG and Mitchell GF, 1996, Immunol. and Cell Biol. **74:** 8-25 (both incorporated by reference herein) provide useful instructions for the preparation of complete immunostimulating complexes.

Another highly interesting (and thus, preferred) possibility of achieving adjuvant effect is to employ the technique described in Gosselin *et al*., 1992 (which is hereby incorporated by reference herein). In brief, the presentation of a relevant antigen such as an antigen of the present invention can be enhanced by conjugating the antigen to antibodies (or antigen binding antibody fragments) against the Fcγ receptors on monocytes/macrophages. Especially conjugates between antigen and anti-FcγRI have been demonstrated to enhance immunogenicity for the purposes of vaccination.

Other possibilities involve the use of the targeting and immune modulating substances (i.a. cytokines) mentioned above as candidates for the first and second moieties in the modified versions of amyloidogenic polypeptides. In this connection, also synthetic inducers of cytokines like poly I:C are possibilities.

Suitable mycobacterial derivatives are selected from the group consisting of muramyl dipeptide, complete Freund's adjuvant, RIBI, and a diester of trehalose such as TDM and TDE.

Suitable immune targeting adjuvants are selected from the group consisting of CD40 ligand and CD40 antibodies or specifically binding fragments thereof (cf. the discussion above), mannose, a Fab fragment, and CTLA-4.

Suitable polymer adjuvants are selected from the group consisting of a carbohydrate such as dextran, PEG, starch, mannan, and mannose; a plastic polymer such as; and latex such as latex beads.

Yet another interesting way of modulating an immune response is to include the immunogen (optionally together with adjuvants and pharmaceutically acceptable carriers and vehicles) in a "virtual lymph node" (VLN) (a proprietary medical device developed by ImmunoTherapy, Inc., 360 Lexington Avenue, New York, NY 10017-6501). The VLN (a thin tubular device) mimics the structrue and function of a lymph node. Insertion of a VLN under the skin creates a site of sterile inflammation with an upsurge of cytokines and chemokines. T- and B-cells as well as APCs rapidly respond to the danger signals, home to the inflamed site and accumulate inside the porous matrix of the VLN. It has been shown that the necessary antigen dose required to mount an immune response to an antigen is reduced when using the VLN and that immune protection conferred by vaccination using a VLN surpassed conventional immunization using Ribi as an adjuvant. The technology is i.a. described briefly in Gelber C *et al.,* 1998, "Elicitation of Robust Cellular and Humoral Immune Responses to Small Amounts of Immunogens Using a Novel Medical Device Designated the Virtual Lymph Node", in: "From the Laboratory to the Clinic, Book of Abstracts, October 12$^{th}$ - 15$^{th}$ 1998, Seascape Resort, Aptos, California".

Microparticle formulation of vaccines has been shown in many cases to increase the immunogenicity of protein antigens and is therefore another preferred embodiment of the invention. Microparticles are made either as co-formulations of antigen with a polymer, a lipid, a carbohydrate or other molecules suitable for making the particles, or the microparticles can be homogeneous particles consisting of only the antigen itself.

Examples of polymer based microparticles are PLGA and PVP based particles (Gupta, R.K. et. al. 1998) where the polymer and the antigen are condensed into a solid particle. Lipid based particles can be made as micelles of the lipid (so-called liposomes) entrapping the antigen within the micelle (Pietrobon, P.J. 1995). Carbohydrate based particles are typically made of a suitable degradable carbohydrate such as starch or chitosan. The carbohydrate and the antigen are mixed and condensed into particles in a process similar to the one used for polymer particles (Kas, H. S. et. al. 1997).

Particles consisting only of the antigen can be made by various spraying and freeze-drying techniques. Especially suited for the purposes of the present invention is the super critical fluid technology that is used to make very uniform particles of controlled size (York, P. 1999 & Shekunov, B. et. al. 1999).

It is expected that the vaccine should be administered 1-6 times per year, such as 1, 2, 3, 4, 5, or 6 times a year to an individual in need thereof. It has previously been shown that the memory immunity induced by the use of the preferred autovaccines according to the invention is not permanent, and therefore the immune system needs to be periodically challenged with the amyloidogenic polypeptide or modified amyloidogenic polypeptides.

Due to genetic variation, different individuals may react with immune responses of varying strength to the same polypeptide. Therefore, the vaccine according to the invention may comprise several different polypeptides in order to increase the immune response, cf. also the discussion above concerning the choice of foreign T-cell epitope introductions. The vaccine may comprise two or more polypeptides, where all of the polypeptides are as defined above.

The vaccine may consequently comprise 3-20 different modified or unmodified polypeptides, such as 3-10 different polypeptides.

<u>Nucleic acid vaccination</u>

As an alternative to classic administration of a peptide-based vaccine, the technology of nucleic acid vaccination (also known as "nucleic acid immunisation", "genetic immunisation", and "gene immunisation") offers a number of attractive features.

First, in contrast to the traditional vaccine approach, nucleic acid vaccination does not require resource consuming large-scale production of the immunogenic agent (e.g. in the form of industrial scale fermentation of microorganisms producing modified amyloidogenic polypeptides). Furthermore, there is no need to device purification and refolding schemes for the immunogen. And finally, since nucleic acid vaccination relies on the biochemical apparatus of the vaccinated individual in order to produce the expression product of the nucleic acid introduced, the optimum post-translational processing of the expression product is expected to occur; this is especially important in the case of autovaccination, since, as mentioned above, a significant fraction of the original B-cell epitopes should be preserved in the modified molecule, and since B-cell epitopes in principle can be constituted by parts of any (bio)molecule (e.g. carbohydrate, lipid, protein etc.). Therefore, native glycosylation and lipidation patterns of the immunogen may very well be of importance for the overall immunogenicity and this is best ensured by having the host producing the immunogen.

Hence, a preferred embodiment of the invention comprises effecting presentation of modified amyloidogenic polypeptide to the immune system by introducing nucleic acid(s) encoding the modified amyloidogenic polypeptide into the animal's cells and thereby obtaining *in vivo* expression by the cells of the nucleic acid(s) introduced.

In this embodiment, the introduced nucleic acid is preferably DNA which can be in the form of naked DNA, DNA formulated with charged or uncharged lipids, DNA formulated in liposomes, DNA included in a viral vector, DNA formulated with a transfection-facilitating protein or polypeptide, DNA formulated with a targeting protein or polypeptide, DNA formulated with Calcium precipitating agents, DNA coupled to an inert carrier molecule, DNA encapsulated in a polymer, e.g. in PLGA (cf. the microencapsulation technology described in WO 98/31398) or in chitin or chitosan, and DNA formulated with an adjuvant. In this context it is noted that practically all considerations pertaining to the use of adjuvants in traditional vaccine formulation apply for the formulation of DNA vaccines. Hence, all disclosures herein which relate to use of adjuvants in the context of polypeptide based vaccines apply *mutatis mutandis* to their use in nucleic acid vaccination technology.

As for routes of administration and administration schemes of polypeptide based vaccines which have been detailed above, these are also applicable for the nucleic acid vaccines of the invention and all discussions above pertaining to routes of administration and administration schemes for polypeptides apply *mutatis mutandis* to nucleic acids. To this should be added that nucleic acid vaccines can suitably be administered intraveneously and intraarterially. Furthermore, it is well-known in the art that nucleic acid vaccines can be administered by use of a so-called gene gun, and hence also this and equivalent modes of administration are regarded as part of the present invention. Finally, also the use of a VLN in the administration of nucleic acids has been reported to yield good results, and therefore this particular mode of administration is particularly preferred.

Furthermore, the nucleic acid(s) used as an immunization agent can contain regions encoding the 1$^{st}$, 2$^{nd}$ and/or 3$^{rd}$ moieties, e.g. in the form of the immunomodulating substances described above such as the cytokines discussed as useful adjuvants. A preferred version of this embodiment encompasses having the coding region for the analogue and the coding region for the immunomodulator in different reading frames or at least under the control of different promoters. Thereby it is avoided that the analogue or epitope is produced as a fusion partner to the immunomodulator. Alternatively, two distinct nucleotide fragments can be used, but this is less preferred because of the advantage of ensured co-expression when having both coding regions included in the same molecule.

Accordingly, the invention also relates to a composition for inducing production of antibodies against an amyloidogenic polypeptide, the composition comprising

- a nucleic acid fragment or a vector of the invention (cf. the discussion of vectors below), and

- a pharmaceutically and immunologically acceptable vehicle and/or carrier and/or adjuvant as discussed above.

Under normal circumstances, the variant-encoding nucleic acid is introduced in the form of a vector wherein expression is under control of a viral promoter. For more detailed discussions of vectors according to the invention, cf. the discussion below. Also, detailed disclosures relating to the formulation and use of nucleic acid vaccines are available, cf. Donnelly JJ *et al*, 1997, Annu. Rev. Immunol. **15:** 617-648 and Donnelly JJ *et al*., 1997, Life Sciences **60:** 163-172. Both of these references are incorporated by reference herein.

Live vaccines

A third alternative for effecting presentation of modified amyloidogenic polypeptide to the immune system is the use of live vaccine technology. In live vaccination, presentation to the immune system is effected by administering, to the animal, a non-pathogenic microorganism which has been transformed with a nucleic acid fragment encoding a modified amyloidogenic polypeptide or with a vector incorporating such a nucleic acid fragment. The non-pathogenic microorganism can be any suitable attenuated bacterial strain (attenuated by means of passaging or by means of removal of pathogenic expression products by recombinant DNA technology), e.g. *Mycobacterium bovis* BCG., non-pathogenic *Streptococcus* spp., *E. coli, Salmonella* spp., *Vibrio cholerae, Shigella,* etc. Reviews dealing with preparation of state-of-the-art live vaccines can e.g. be found in Saliou P, 1995, Rev. Prat. **45**: 1492-1496 and Walker PD, 1992, Vaccine **10:** 977-990, both incorporated by reference herein. For details about the nucleic acid fragments and vectors used in such live vaccines, cf. the discussion below.

As an alternative to bacterial live vaccines, the nucleic acid fragment of the invention discussed below can be incorporated in a non-virulent viral vaccine vector such as a vaccinia strain or any other suitable pox virus.

Normally, the non-pathogenic microorganism or virus is administered only once to the animal, but in certain cases it may be necessary to administer the microorganism more than once in a lifetime in order to maintain protective immunity. It is even contemplated that immunization schemes as those detailed above for polypeptide vaccination will be useful when using live or virus vaccines.

Alternatively, live or virus vaccination is combined with previous or subsequent polypeptide and/or nucleic acid vaccination. For instance, it is possible to effect primary immunization with a live or virus vaccine followed by subsequent booster immunizations using the polypeptide or nucleic acid approach.

The microorganism or virus can be transformed with nucleic acid(s) containing regions encoding the 1$^{st}$, 2$^{nd}$ and/or 3$^{rd}$ moieties, e.g. in the form of the immunomodulating substances described above such as the cytokines discussed as useful adjuvants. A preferred version of this embodiment encompasses having the coding region for the analogue and the coding region for the immunomodulator in different reading frames or at least under the control of different promoters. Thereby it is avoided that the analogue or epitopes are produced as fusion partners to the immunomodulator. Alternatively, two distinct nucleotide fragments can be used as transforming agents. Of course, having the 1$^{st}$ and/or 2$^{nd}$ and/or 3$^{rd}$ moieties in the same reading frame can provide as an expression product, an analogue of the invention, and such an embodiment is especially preferred according to the present invention.

Use of the method of the invention in disease treatment

As will be appreciated from the discussions above, the provision of the method of the invention allows for control of diseases characterized by amyloid deposits. In this context, AD is the key target for the inventive method but also other diseases characterized by amyloid deposits are feasible targets. Hence, an important embodiment of the method of the invention for down-regulating amyloid activity comprises treating and/or preventing and/or ameliorating AD or other diseases characterized by amyloid deposition, the method comprising down-regulating amyloid according to the method of the invention to such an extent that the amount of amyloid is significantly decreased.

It is especially preferred that the reduction in amyloid results in an inversion of the balance between amyloid formation and amyloid degradation/removal, i.e. that the rate of amyloid degradation/removal is brought to exceed the rate of amyloid formation. By carefully controlling the number and immunological impact of immunizations of the individual in need thereof it will be possible to obtain a balance over time which results in a net reduction of amyloid deposits without having excessive adverse effects.

Alternatively, if in an individual the method of the invention cannot remove or reduce existing amyloid deposits, the method of the invention can be used to obtain a clinically significant reduction in the formation of new amyloid, thereby significantly prolonging the time where the disease condition is non-debilitating. It should be possible to monitor the rate of amyloid depositing by either measuring the serum concentration of amyloid (which is believed to be in equilibrium with the deposited material), or by using positron-emission tomography (PET) scanning, cf. Small GW, *et al*., 1996, Ann N Y Acad Sci 802: 70-78.

Other diseases and conditions where the present means and methods may be used in treatment or amelioration in an analogous way have been mentioned above in the "Background of the invention" (systemic amyloidosis, maturity onset diabetes, Parkinson's disease, Huntington's disease, fronto-temporal dementia and the prion-related transmissible spongiform encephalopathies) or are listed below in the section headed "other amyloidic diseases and proteins associated therewith".

Peptides, polypeptides, and compositions of the invention

As will be apparent from the above, the present invention is based on the concept of immunising individuals against the amyloidogenic antigen in order to obtain a reduced amount of pathology-related amyloid deposits. The preferred way of obtaining such an immunization is to use modified versions of amyloidogenic polypeptide, thereby providing molecules which have not previously been disclosed in the art.

It is believed that the modified molecules discussed herein are inventive in their own right, and therefore an important part of the invention pertains to an analogue of an amyloidogenic polypeptide which is derived from an animal's autologous Aβ or APP wherein is introduced at least one isolated foreign $T_H$ epitope as schematically shown for the P2 and P30 epitopes in Fig. 1, so that Immunization of the animal with the analogue induces production of antibodies against the amyloidogenic polypeptide. Preferably, the nature of the modification conforms with the types of modifications described above when discussing various embodiments of the method of the invention when using modified amyloidogenic polypeptide. Hence, any disclosure presented herein pertaining to modified amyloidogenic molecules are relevant for the purpose of describing the amyloidogenic analogues of the invention, and any such disclosures apply *mutatis mutandis* to the description of these analogues.

It should be noted that preferred modified amyloidogenic molecules comprises modifications which results in a polypeptide having a sequence identity of at least 70% with an amyloidogenic protein or with a subsequence thereof of at least 10 amino acids in length. Higher sequence identities are preferred, e.g. at least 75% or even at least 80, 85, 90, or 95%. The sequence identity for proteins and nucleic acids can be calculated as $(N_{ref} - N_{dif})\cdot 100/N_{ref}$, wherein $N_{dif}$ is the total number of non-identical residues in the two sequences when aligned and wherein $N_{ref}$ is the number of residues in one of the sequences. Hence, the DNA sequence AGTCAGTC will have a sequence identity of 75% with the sequence AATCAATC ($N_{dif}$=2 and $N_{ref}$=8).

The invention also pertains to compositions useful in exercising the method of the invention.

The invention therefore relates to an immunogenic composition comprising an immunologically effective amount of an analogue defined above, said composition further comprising a pharmaceutically and immunologically acceptable diluent and/or vehicle and/or carrier and/or excipient and optionally an adjuvant. In other words, this part of the invention concerns formulations of modified amyloidogenic polypeptide, essentially as described above. The choice of adjuvants, carriers, and vehicles is accordingly in line with what has been discussed above when referring to formulation of modified and unmodified amyloidogenic polypeptide for use in the inventive method for the down-regulation of amyloid.

The polypeptides are prepared according to methods well-known in the art. Longer polypeptides are normally prepared by means of recombinant gene technology including introduction of a nucleic acid sequence encoding the analogue into a suitable vector, transformation of a suitable host cell with the vector, expression by the host cell of the nucleic acid sequence, recovery of the expression product from the host cells or their culture supernatant, and subseqeunt purification and optional further modification, e.g. refolding or derivatization.

Shorter peptides are preferably prepared by means of the well-known techniques of solid- or liquid-phase peptide synthesis. However, recent advances in this technology has rendered possible the production of full-length polypeptides and proteins by these means, and therefore it is also within the scope of the present invention to prepare the long constructs by synthetic means.

Nucleic acid fragments and vectors of the invention

It will be appreciated from the above disclosure that modified amyloidogenic polypeptides can be prepared by means of recombinant gene technology but also by means of chemical synthesis or semisynthesis; the latter two options are especially relevant when the modification consists in coupling to non-proteinaceous molecules such as carbohydrate polymers and of course also when the modification comprises addition of side chains or side groups to an amyloidogenic polypeptide-derived peptide chain.

For the purpose of recombinant gene technology, and of course also for the purpose of nucleic acid immunization, nucleic acid fragments encoding modified amyloidogenic polypeptide are important chemical products. Hence, an important part of the invention pertains to a nucleic acid fragment which encodes an analogue of an amyloidogenic polypeptide, i.e. an amyloidogenic polypeptide-derived polypeptide which either comprises the natural sequence to which has been added or inserted a fusion partner or, preferably an amyloidogenic polypeptide-derived polypeptide wherein has been introduced a foreign T-cell epitope by means of insertion and/or addition, preferably by means of substitution and/or deletion. The nucleic acid fragments of the invention are either DNA or RNA fragments.

The nucleic acid fragments of the invention will normally be inserted in suitable vectors to form cloning or expression vectors carrying the nucleic acid fragments of the invention; such novel vectors are also part of the invention. Details concerning the construction of these vectors of the invention will be discussed in context of transformed cells and microorganisms below. The vectors can, depending on purpose and type of application, be in the form of plasmids, phages, cosmids, mini-chromosomes, or virus, but also naked DNA which is only expressed transiently in certain cells is an important vector. Preferred cloning and expression vectors of the invention are capable of autonomous replication, thereby enabling high copy-numbers for the purposes of high-level expression or high-level replication for subsequent cloning.

The general outline of a vector of the invention comprises the following features in the 5'→3' direction and in operable linkage: a promoter for driving expression of the nucleic acid fragment of the invention, optionally a nucleic acid sequence encoding a leader peptide enabling secretion (to the extracellular phase or, where applicable, into the periplasma) of or integration into the membrane of the polypeptide fragment, the nucleic acid fragment of the invention, and optionally a nucleic acid sequence encoding a terminator. When operating with expression vectors in producer strains or cell-lines it is for the purposes of genetic stability of the transformed cell preferred that the vector when introduced into a host cell is integrated in the host cell genome. In contrast, when working with vectors to be used for effecting *in vivo* expression in an animal (i.e. when using the vector in DNA vaccination) it is for security reasons preferred that the vector is incapable of being integrated in the host cell genome; typically, naked DNA or non-integrating viral vectors are used, the choices of which are well-known to the person skilled in the art

The vectors of the invention are used to transform host cells to produce the modified amyloidogenic polypeptide of the invention. Such transformed cells, which are also part of the invention, can be cultured cells or cell lines used for propagation of the nucleic acid fragments and vectors of the invention, or used for recombinant production of the modified amyloidogenic polypeptides of the invention. Alternatively, the transformed cells can be suitable live vaccine strains wherein the nucleic acid fragment (one single or multiple copies) have been inserted so as to effect secretion or integration into the bacterial membrane or cell-wall of the modified amyloidogenic polypeptide.

Preferred transformed cells of the invention are microorganisms such as bacteria (such as the species *Escherichia* [e.g. *E.coli*], *Bacillus* [e.g. *Bacillus subtilis*], *Salmonella,* or *Mycobacterium* [preferably non-pathogenic, e.g. *M. bovis* BCG]), yeasts (such as *Saccharomyces cerevisiae),* and protozoans. Alternatively, the transformed cells are derived from a multicellular organism such as a fungus, an insect cell, a plant cell, or a mammalian cell. Most preferred are cells derived from a human being, cf. the discussion of cell lines and vectors below. Recent results have shown great promise in the use of a commercially available *Drosophila melanogaster* cell line (the Schneider 2 ($S_2$) cell line and vector system available from Invitrogen) for the recombinant production of polypeptides in applicants' lab, and therefore this expression system is particularly preferred.

For the purposes of cloning and/or optimized expression it is preferred that the transformed cell is capable of replicating the nucleic acid fragment of the invention. Cells expressing the nucleic fragment are preferred useful embodiments of the invention; they can be used for small-scale or large-scale preparation of the modified amyloidogenic polypeptide or, in the case of non-pathogenic bacteria, as vaccine constituents in a live vaccine.

When producing the modified molecules of the invention by means of transformed cells, it is convenient, although far from essential, that the expression product is either exported out into the culture medium or carried on the surface of the transformed cell.

When an effective producer cell has been identified it is preferred, on the basis thereof, to establish a stable cell line which carries the vector of the invention and which expresses the nucleic acid fragment encoding the modified amyloidogenic polypeptide. Preferably, this stable cell line secretes or carries the analogue of the invention, thereby facilitating purification thereof.

In general, plasmid vectors containing replicon and control sequences which are derived from species compatible with the host cell are used in connection with the hosts. The vector ordinarily carries a replication site, as well as marking sequences which are capable of providing phenotypic selection in transformed cells. For example, *E. coli* is typically transformed using pBR322, a plasmid derived from an *E. coli* species (see, e.g., Bolivar *et al.*, 1977). The pBR322 plasmid contains genes for ampicillin and tetracycline resistance and thus provides easy means for identifying transformed cells. The pBR plasmid, or other microbial plasmid or phage must also contain, or be modified to contain, promoters which can be used by the prokaryotic microorganism for expression.

Those promoters most commonly used in recombinant DNA construction include the B-lactamase (penicillinase) and lactose promoter systems (Chang *et al.*, 1978; Itakura *et al.*, 1977; Goeddel *et al.*, 1979) and a tryptophan (trp) promoter system (Goeddel *et al*., 1979; EP-A-0 036 776). While these are the most commonly used, other microbial promoters have been discovered and utilized, and details concerning their nucleotide sequences have been published, enabling a skilled worker to ligate them functionally with plasmid vectors (Siebwenlist *et al*., 1980). Certain genes from prokaryotes may be expressed efficiently in *E. coli* from their own promoter sequences, precluding the need for addition of another promoter by artificial means.

In addition to prokaryotes, eukaryotic microbes, such as yeast cultures may also be used, and here the promoter should be capable of driving expression. *Saccharomyces cerevisiase,* or common baker's yeast is the most commonly used among eukaryotic microorganisms, although a number of other strains are commonly available. For expression in *Saccharomyces,* the plasmid YRp7, for example, is commonly used (Stinchcomb *et al*., 1979; Kingsman *et al.,* 1979; Tschemper *et al*., 1980). This plasmid already contains the trpl gene which provides a selection marker for a mutant strain of yeast lacking the ability to grow in tryptophan for example ATCC No. 44076 or PEP4-1 (Jones, 1977). The presence of the trpl lesion as a characteristic of the yeast host cell genome then provides an effective environment for detecting transformation by growth in the absence of tryptophan.

Suitable promoting sequences in yeast vectors include the promoters for 3-phosphoglycerate kinase (Hitzman *et al.,* 1980) or other glycolytic enzymes (Hess *et al*., 1968; Holland et *al*., 1978), such as enolase, glyceraldehyde-3-phosphate dehydrogenase, hexokinase, pyruvate decarboxylase, phosphofructokinase, glucose-6-phosphate isomerase, 3-phosphoglycerate mutase, pyruvate kinase, triosephosphate isomerase, phosphoglucose isomerase, and glucokinase. In constructing suitable expression plasmids, the termination sequences associated with these genes are also ligated into the expression vector 3' of the sequence desired to be expressed to provide polyadenylation of the mRNA and termination.

Other promoters, which have the additional advantage of transcription controlled by growth conditions are the promoter region for alcohol dehydrogenase 2, isocytochrome C, acid phosphatase, degradative enzymes associated with nitrogen metabolism, and the aforementioned glyceraldehyde-3-phosphate dehydrogenase, and enzymes responsible for maltose and galactose utilization. Any plasmid vector containing a yeast-compatible promoter, origin of replication and termination sequences is suitable.

In addition to microorganisms, cultures of cells derived from multicellular organisms may also be used as hosts. In principle, any such cell culture is workable, whether from vertebrate or invertebrate culture. However, interest has been greatest in vertebrate cells, and propagation of vertebrate in culture (tissue culture) has become a routine procedure in recent years (Tissue Culture, 1973). Examples of such useful host cell lines are VERO and HeLa cells, Chinese hamster ovary (CHO) cell lines, and W138, BHK, COS-7 293, *Spodoptera frugiperda* (SF) cells (commercially available as complete expression systems from *i.a*. Protein Sciences, 1000 Research Parkway, Meriden, CT 06450, U.S.A. and from Invitrogen), and MDCK cell lines. In the present invention, an especially preferred cell line is $S_2$ available from Invitrogen, PO Box 2312, 9704 CH Groningen, The Netherlands.

Expression vectors for such cells ordinarily include (if necessary) an origin of replication, a promoter located in front of the gene to be expressed, along with any necessary ribosome binding sites, RNA splice sites, polyadenylation site, and transcriptional terminator sequences.

For use in mammalian cells, the control functions on the expression vectors are often provided by viral material. For example, commonly used promoters are derived from polyoma, Adenovirus 2, and most frequently Simian Virus 40 (SV40). The early and late promoters of SV40 virus are particularly useful because both are obtained easily from the virus as a fragment which also contains the SV40 viral origin of replication (Fiers *et al*., 1978). Smaller or larger SV40 fragments may also be used, provided there is included the approximately 250 bp sequence extending from the HindIII site toward the *Bgl*I site located in the viral origin of replication. Further, it is also possible, and often desirable, to utilize promoter or control sequences normally associated with the desired gene sequence, provided such control sequences are compatible with the host cell systems.

An origin of replication may be provided either by construction of the vector to include an exogenous origin, such as may be derived from SV40 or other viral (e.g., Polyoma, Adeno, VSV, BPV) or may be provided by the host cell chromosomal replication mechanism. If the vector is integrated into the host cell chromosome, the latter is often sufficient.

Identification of useful analogues

It will be clear to the skilled person that not all possible variants or modifications of naturally occurring amyloidogenic polypeptides will have the ability to elicit antibodies in an animal which are cross-reactive with the natural form. It is, however, not difficult to set up an effective standard screen for modified amyloidogenic molecules which fulfill the minimum requirements for immunological reactivity discussed herein. Hence, one method for the identification of a modified amyloidogenic polypeptide which is capable of inducing antibodies against unmodified amyloidogenic polypeptide in an animal species where the unmodified amyloidogenic polypeptide is a (non-immunogenic) self-protein, comprises

- preparing, by means of peptide synthesis or genetic engineering techniques, a set of mutually distinct modified amyloidogenic polypeptides wherein amino acids have been added to, inserted in, deleted from, or substituted into the amino acid sequence of an amyloidogenic polypeptide of the animal species thereby giving rise to amino acid sequences in the set which comprise T-cell epitopes which are foreign to the animal species, or preparing a set of nucleic acid fragments encoding the set of mutually distinct modified amyloidogenic polypeptides,
- testing members of the set of modified amyloidogenic polypeptides or nucleic acid fragments for their ability to induce production of antibodies by the animal species against the unmodified amyloidogenic polypeptide, and
- identifying and optionally isolating the member(s) of the set of modified amyloidogenic polypeptides which significantly induces antibody production against unmodified amyloidogenic polypeptide in the species or identifying and optionally isolating the polypeptide expression products encoded by members of the set of nucleic acid fragments which significantly induces antibody production against unmodified amyloidogenic polypeptide in the animal species.

In this context, the "set of mutually distinct modified amyloidogenic polypeptides" is a collection of non-identical modified amyloidogenic polypeptides which have e.g. been selected on the basis of the criteria discussed above (e.g. in combination with studies of circular dichroism, NMR spectra, and/or X-ray diffraction patterns). The set may consist of only a few members but it is contemplated that the set may contain several hundred members.

The test of members of the set can ultimately be performed *in vivo,* but a number of in vitro tests can be applied which narrow down the number of modified molecules which will serve the purpose of the invention.

Since the goal of introducing the foreign T-cell epitopes is to support the B-cell response by T-cell help, a prerequisite is that T-cell proliferation is induced by the modified amyloidogenic polypeptide. T-cell proliferation can be tested by standardized proliferation assays in vitro. In short, a sample enriched for T-cells is obtained from a subject and subsequently kept in culture. The cultured T-cells are contacted with APCs of the subject which have previously taken up the modified molecule and processed it to present its T-cell epitopes. The proliferation of T-cells is monitored and compared to a suitable control (e.g. T-cells in culture contacted with APCs which have processed intact, native amyloidogenic polypeptide). Alternatively, proliferation can be measured by determining the concentration of relevant cytokines released by the T-cells in response to their recognition of foreign T-cells.

Having rendered highly probable that at least one modified amyloidogenic polypeptide of either type of set is capable of inducing antibody production against amyloidogenic polypeptide, it is possible to prepare an immunogenic composition comprising at least one modified amyloid polypeptide which is capable of inducing antibodies against unmodified amyloidogenic polypeptide in an animal species where the unmodified amyloidogenic polypeptide is a self-protein, the method comprising admixing the member(s) of the set which significantly induces production of antibodies in the animal species which are reactive with the amyloidogenic polypeptide with a pharmaceutically and immunologically acceptable carrier and/or vehicle and/or diluent and/or excipient, optionally in combination with at least one pharmaceutically and immunologically acceptable adjuvant.

The above methods pertaining to test of polypeptide sets are conveniently carried out by initially preparing a number of mutually distinct nucleic acid sequences or vectors of the invention, inserting these into appropriate expression vectors, transforming suitable host cells (or host animals) with the vectors, and effecting expression of the nucleic acid sequences of the invention. These steps can be followed by isolation of the expression products. It is preferred that the nucleic acid sequences and/or vectors are prepared by methods comprising exercise of a molecular amplification technique such as PCR or by means of nucleic acid synthesis.

Other amyloidic diseases and proteins associated therewith

There are a relatively large number of diseases other than AD where amyloid formation is involved in triggering the disease or in causing the symptoms of the disease. Although the.proteins involved in these diseases vary in nature they share the same features which define amyloid, cf. above. The following table lists a number of these amyloidic disorders and the proteins causing them.

| Diversity of amyloid fibril proteins | | |
|---|---|---|
| Clinical Syndrome | Fibril subunit | Precursor structure |
| Cerebral amyloid angiopathy (CAA) | Aβ | All β |
| Monoclonal protein systemic (AL) amyloidosis | Full length or fragments of ν domain of IG light chain | All β |
| Reactive systemic (AA) amyloidosis | 76-residue N-terminal fragment of amyloid A protein | α/β |
| Familial amyloidotic polyneuropathy | Full-length or fragments of transthyretin variants | All β |
| Hereditary ApoAI amyloidosis | N-terminal fragments (-90 residues) of ApoAI variants | (α/β) |
| Hereditary lysozyme amyloidosis | Full-length lysozyme variants | α + β |
| Type II diabetes mellitus | 37-residue fragment of islet-amyloid polypeptide | Unknown |
| Insulin-related amyloid | Full-length wild-type insulin | a + β |
| Transmissible spongioform encephalopathis | Full-length or fragments of prion protein | α + β |
| Medullary carcinoma of the thyroid | Fragments of calcitonin | Unknown |
| Senile systemic amyloidosis | Full-length or fragments of transthyretin | All β |
| Hemodialysis-related amyloidosis | Full-length, wild-type β-2 microglobulin | All P |
| Isolated atrial amyloidosis | Atrial natriuretic factor | Unknown |
| Hereditary cerebral amyloid angiopathy | 110-residue fragment of variant cystatin | α + β |
| Finnish hereditary amyloidosis | 71-residue fragment of gelsolin variants | α/β |
| Hereditary fibrinogen a-chain amyloidosis | Fragments of fibrinogen a-chain variants | Unknown |

It is contemplated that most methods for immunizing against amyloidogenic polypeptides should be restricted to immunization giving rise to antibodies cross-reactive with the native amyloidogenic polypeptide. Nevertheless, in some cases it will be of interest to induce cellular immunity in the form of CTL responses against cells which present MHC Class I epitopes from the amyloidogenic polypeptides - this can be expedient in those cases wherein reduction in the number of cells producing the amyloidogenic polypeptides does not constitute a serious adverse effect. In such cases where CTL responses are desired it is preferred to utilise the teachings of Applicant's PCT/DK99/00525 (corresponding to USSN 09/413,186). The disclosures of these two documents are hereby incorporated by reference herein.

In the following non-limiting example, focus has been put on the development of a Aβ based autovaccine against AD.

EXAMPLE 1

*The Auto Vaccination approach for Immunizing against AD*

The fact that Aβ protein knock out mice does not show any abnormalities or adverse side effects, suggest that removal or lowering the amounts of Aβ will be safe, Zheng H. (1996).

Published experiments where transgenic animals are immunized against the transgenic human Aβ protein suggest that if it was possible to break the selt tolerance down regulation of Aβ could be obtained by auto-reactive antibodies. These experiments further suggest that such down regulation of Aβ potentially would both prevent the formation of plaques, and even clear already formed Aβ plaques from the brain, cf. Schenk et al. (1999). But, traditionally it is not possible to raise antibodies against self-proteins.

The published data does thus not provide the means for breaking true self-tolerance towards true self-proteins. Nor does the data provide information on how to ensure that the immune reaction is directed solely or predominantly towards the Aβ deposits, and not towards the cell membrane bound Aβ precursor protein (APP), if this is deemed necessary. An immune response generated using the existing technology would presumably generate an immune response towards self-proteins in an unregulated way so unwanted and excessive auto-reactivity towards parts the Aβ protein may be generated. Hence, using existing immunization strategies will most likely be unable to generate strong immune responses towards self-proteins and will furthermore be unsafe due to potential strong cross-reactivity towards membrane bound APP which is present on a large number of cells in the CNS.

The present invention provides the means of effectively generating a strong regulated immune response towards true self-proteins which potentially could form plaques and cause serious disease in the CNS or in other compartments of the body. A safe and efficacious human Aβ protein therapeutic vaccine will be developed by using this technology for the treatment of AD.

In light of this, it is possible to anticipate that AD, a disease predicted to cripple the health care system in the next century, could be cured, or such vaccines described could at least constitute an effective therapeutical approach for treatment of the symptoms and progression of this disease.

This technique represents a entirely new immunological approach to blocking amyloid deposition in AD and other neurologic diseases as well.

In the following table, 35 contemplated constructs are indicated. All positions given in the table are relative to the starting Methionine of APP (first amino acid in SEQ ID NO: 2) and include both the starting and ending amino acid, e.g. the 672 - 714 fragment includes both amino acid 672 and 714. The starting and ending positions for P2 and P30 indicate that the epitope substitutes a part of the APP fragment at the positions indicated (both positions included in the substitution) - in most constructs, the introduced epitopes substitutes a fragment of the length of the epitope. The asterisks in the table have the following meaning:

*) Only one position for P2 and P30 indicates that the epitope has been inserted into the APP derivative at the position indicated (the epitope begins at the amino acid C-terminally adjacent to the given position).

**) Construction 34 contains three identical APP fragments separated by P30 and P2, respectively.

***) Construction 35 contains nine identical APP fragments separated by alternating P30 and P2 epitopes.

APP AutoVac constructions

| Var. No. | Start of APP segment relative to aa 1 of APP | End of APP segment relative to aa 1 of APP | Position of P2 epitope relative to aa 1 of APP | Position of P30 epitope relative to aa 1 of APP | Molecule length |
|---|---|---|---|---|---|
| 1 | 630 | 770 | 656 - 670 | 635 - 655 | 141 |
| 2 | 630 | 714 | 656 - 670 | 635 - 655 | 85 |
| 3 | 672 | 770 | 735 - 749 | 714 - 728 | 99 |
| 4 | 672 | 770 | | 714 - 728 | 99 |
| 5 | 672 | 770 | 714 - 728 | | 99 |
| 6 | 672 | 770 | 723* | 723* | 135 |
| 7 | 672 | 770 | | 723* | 120 |
| 8 | 672 | 770 | 723* | | 114 |
| 9 | 672 | 714 | | 672* | 64 |
| 10 | 672 | 714 | | 714* | 64 |
| 11 | 672 | 714 | 672* | | 58 |
| 12 | 672 | 714 | 714* | | 58 |
| 13 | 672 | 714 | 714* | 672* | 79 |
| 14 | 672 | 714 | 680 - 694 | | 43 |
| 14 | 672 | 714 | 685 - 799 | | 43 |
| 16 | 672 | 714 | 690 - 704 | | 43 |
| 17 | 672 | 714 | 695 - 709 | | 43 |
| 18 | 672 | 714 | | 675 - 695 | 43 |
| 19 | 672 | 714 | | 680 - 700 | 43 |
| 20 | 672 | 714 | | 685 - 705 | 43 |
| 21 | 672 | 714 | | 690 - 710 | 43 |
| 22 | 672 | 714 | 680* | 680* | 79 |
| 23 | 672 | 714 | 690* | 690* | 79 |
| 24 | 672 | 714 | 700* | 700* | 79 |
| 25 | 672 | 714 | 710* | 710* | 79 |
| 26 | 672 | 714 | | 680* | 64 |
| 27 | 672 | 714 | | 690* | 64 |
| 28 | 672 | 714 | | 700* | 64 |
| 29 | 672 | 714 | | 710* | 64 |
| 30 | 672 | 714 | 680* | | 58 |
| 31 | 672 | 714 | 690* | | 58 |
| 32 | 672 | 714 | 700* | | 58 |
| 33 | 672 | 714 | 710* | | 58 |
| 34 | 672 | 714 | After rep. 1** | After rep. 2** | 165 |
| 35 | 672 | 714 | 34 x 3* | 34 x 3*** | 165 |

The part of APP against which it most interesting to generate a response is the 43 amino acid Aβ core peptide (Aβ-43, corresponding to SEQ ID NO: 2, residues 672-714) that is the main constituent of amyloid plaques in AD brains. This APP fragment is part of all constructions listed above.

Variants 1 and 2 comprise a portion of APP upstream of Aβ-43 where the model epitopes P2 and P30 have been placed. Variants 1 and 3-8 all comprise the C-100 fragment which has been shown to be neurotoxic - the C-100 fragment corresponds to amino acid residues 714-770 of SEQ ID NO: 2. In variants 3-5 the epitopes substitutes a part of the C-100 fragment while the in variants 6-8 have been inserted into C-100.

Variants 9-35 contain only the core Aβ-43 protein. In variants 9-13, P2 and P30 are fused to either end of Aβ-43; in 14-21 P2 and P30 substitutes part of Aβ-43; in 22-33 P2 and P30 are inserted into Aβ-43; 34 contains three identical Aβ-93 fragments spaced by P30 and P2, respectively; 35 contains 9 Aβ-43 repeats spaced by alternating P2 and P30 epitopes.

See Fig. 1 and the table above for details.

One further type of construct is especially preferred. Since one goal of the present invention is to avoid destruction of the cells producing APP whereas removal of Aβ is desired, it seems feasible to prepare autovaccine constructs comprising only parts of Aβ which are not exposed to the extracellular phase when present in APP. Thus, such constructs would need to contain at least one B-cell epitope derived from the amino acid fragment defined by amino acids 700-714 in SEQ ID NO: 2.

Since such a short polypeptide fragment is predicted to be only weakly immunogenic it is preferred that such an autovaccine construct consists of several copies of the B-cell epitope, e.g. in the form of a construct having the structure shown in Formula I in the detailed disclosure of the present invention, cf. above. In that version of Formula I, the terms $amyloid_{e1}$-$amyloid_{ex}$ are x B-cell epitope containing amino acid sequences derived from amino acids 700-714 of SEQ ID NO: 2. A preferred alternative is the above-detailed possibility of coupling the amyloidogenic (poly) peptide and the selected foreign T-helper epitope to via an amide bond to a polysaccharide carrier molecule - in this way multiple presentaions of the "weak" epitope constituted by amino acids 700-714 of SEQ ID NO: 2 become possible, and it also becomes possible to select an optimum ratio between B-cell and T-cell epitopes.

EXAMPLE 2

*Immunisation of transgenic mice with Aβ and modified proteins according to the invention*

**Construction of the hAB43+-34 encoding DNA.** The hAB43+-34 gene was constructed in several steps. First a PCR fragment was generated with primers ME#801 (SEQ ID NO: 10) and ME#802 (SEQ ID NO: 11) using primer ME#800 (SEQ ID NO: 9) as template. ME#800 encodes the human abeta-43 fragment with E. coli optimised codons. ME#801 and 802 adds appropriate restriction sites to the fragment.

The PCR fragment was purified, digested with *Nco*I and HindIII, purified again and cloned into NcoI-HindIII digested and purified pET28b+ *E. coli* expression vector. The resulting plasmid encoding wildtype human Aβ-43 is named pAB1.

In the next step the T-helper epitope, P2, is added to the C-terminus of the molecule. Primer ME#806 (SEQ ID NO: 12) contains the sequence encoding the P2 epitope, thus generating a fusion of P2 and Abeta-43 by the PCR reaction.

The cloning was performed by making a PCR fragment with primers ME#178 (SEQ ID NO: 8) and ME#806 using pAB1 as template. The fragment was purified, digested with *Nco*I and HindIII, purified again and cloned into an NcoI-HindIII digested and purified pET28b+ vector. The resulting plasmid is called pAB2.

In an analogous manner, another plasmid was made harbouring the Aβ-43 encoding sequence with another T helper epitope, P30, added to the N-terminus. This was done by making a PCR fragment with primers ME#105 (SEQ ID NO: 7) and ME#807 (SEQ ID NO: 13) using pAB1 as template.

The fragment was purified, digested with *Nco*I and *Hind*III, purified again and cloned into an *Nco*I-*Hind*III digested and purified pET28b+ vector. The resulting plasmid is called pAB3.

In the third step, a second Aβ-43 repeat is added C-terminally to the P2 epitope of plasmid pAB2 by primer ME#809 (SEQ ID NO: 14). ME#809 at the same time creates a *Bam*HI site immediately after the Aβ-43 repeat. A PCR fragment was made with primers ME#178 and ME#809 using pAB2 as template. The fragment was digested with *Nco*I and *Hind*III, purified and cloned into NcoI-HindIII digested and purified pET28b+ vector. This plasmid is named pAB4.

Finally, the P30 epitope - Aβ-43 repeat sequence from pAB3 was cloned into pAB4 plasmid. This was done by making a PCR fragment with primers ME#811 (SEQ ID NO: 16) and ME#105 using pAB3 as template. The fragment was purified and used as primer in a subsequent PCR with ME#810 (SEQ ID NO: 15) using pAB3 as template. The resulting fragment was purified, digested with *Bam*HI and *Hind*III and cloned into *Bam*HI-*Hind*III digested and purified pAB4 plasmid. The resulting plasmid, pAB5, encodes the hAB43+-34 molecule.

All PCR and cloning procedures were done essentially as described by Sambrook, J., Fritsch, E.F. & Maniatis, T. 1989 "Molecular cloning: a laboratory manual". 2nd. Ed. Cold Spring Harbor Laboratory, N.Y.

For all cloning procedures *E. coli* K-12 cells, strain Top-10 F' (Stratagene, USA), were used. The pET28b+ vector was purchased from Novagen, USA. All primers were synthesised at DNA Technology, Denmark.

**Expression and purification of hAB43+-34**. The hAB43+-34 protein encoded by pAB5 was expressed in BL21-Gold (Novagen) *E. coli* cells as described by the suppliers of the pET28b+ system (Novagen).

The expressed hAB43+-34 protein was purified to more than 85% purity by washing of inclusion bodies followed by cation-exchange chromatography using a BioCad purification workstation (PerSeptive Biosystems, USA) in the presence of 6 M urea. The urea was hereafter removed by stepwise dialysis against a solution containing decreasing amounts of urea. The final buffer was 10 mM Tris, pH 8.5.

**Immunisation study.** Mice transgenic for human APP (Alzheimer's precursor protein) were used for the study. These mice, called TgRND8+, express a mutated form of APP that results in high concentration of Aβ-40 and Aβ-42 in the mouse brains (Janus, C. *et. al.*)

The mice (8-10 mice per group) were immunised with either Abeta-42 (SEQ TD NO: 2, residues 673-714, synthesised by means of a standard Fmoc strategy) or the hAB43+-34 variant (construct 34 in the table in Example 1, recombinantly produced) four times at two-week intervals. Doses were either 100 mg for Aβ or 50 mg for hAB43+-34. Mice were bled at day 43 (after three injections) and after day 52 (after four injections) and the sera were used to determine the level of anti-Aβ-42 specific titres using a direct Aβ-42 ELISA.

The following tabel shows the mean relative anti-Abeta-42 titres.

| Immunogen | Day 43 (after 3 immunizations) | Day 52 (after 4 immunizations) |
|---|---|---|
| Aβ-42 | 4000 | 3000 |
| hAB43+-34 | 16000 | 23000 |

As will be clear, the antibody titers obtained when immunizing with the hAB43+-34 Aβ variant are approximately 4 times and 7.5 times higher after 3 and 4 immunizations, respectively, than the titers obtained when using the unaltered wild-type Aβ-42 as an immunogen. This fact is put further in perspective, when considering the fact that the amount of variant used for immunization was only 50% of the amount of wild-type sequence used for immunization.

LIST OF REFERENCES

Brookmeyer., R ; Gray, S., Kavas, C. (1998), Projections of Alzheimer's Disease in the United States and the Public Health Impact of Delaying Disease Onset. American Journal of Public Health, 88(9), 1337-1342.

Buttini, M.; Orth, M.; Bellosta, S.; Akeefe, H.; Pitas, R.E.; Wyss-Coray, T.; Mucke, L.; Mahley, R.W. (1999). Expression of Human Apolipoprotein E3 or E4 in the Brains of Apoe-/- Mice: Isoform-Specific Effects on Neurodegeneration. Journal of Neuroscience, 19, 4867-4880.

Clark, L.N.; Poorkaj, P.; Wszolek, Z.; Geschwind, D.H.; Nasreddine, Z.S.; Miller, B.; Li, D.; Payami, H.; Awert, F.; Markopoulou, K.; Andreadis, A.; D'Souza, I.; Lee, V.M.; Reed, L.; Trojanowski, J.Q.; Zhukareva, V.; Bird, T.; Schellenberg, G.; Wilhelmsen, K.C. (1998). Pathogenic Implications of Mutations in the Tau Gene in Pallido-Ponto-Nigral Degeneration and Related Neurodegenerative Disorders Linked to Chromosome 17. Proceedings of the National Academy of Sciences U.S.A., 95(22), 13103-13107.

Gupta, R. K. *et. al*. (1998), Dev Biol Stand. **92:** 63-78.

Hsiao K. et al. (1998) Transgenic mice expressing Alzheimer amyloid precursor proteins", Exp. Gerontol. 33 (7-8), 883-889

Hutton, M.; Lendon, C.L.; Rizzu, P.; Baker, M.; Froelich, S.; Houlden, H.; Pickering-Brown, S.; Chakraverty, S.; Isaacs, A.; Grover, A.; Hackett, J.; Adamson, J.; Lincoln, S.; Dickson, D.; Davies, P.; Petersen, R.C.; Stevens, M.; de Graaff, E.; Wauters, E.; van Baren, J.; Hillebrand, M.; Joosse, M.; Kwon, J.M.; Nowotny, P.; Che, L.K.; Norton, J.; Morris, J.C.; Reed, L.E.; Trojanowski, J.; Basun, H.; Lannfelt, L.; Neystat, M.; Fahn, S.; Dark, F.; Tannenberg, T.; Dodd, P.; Hayward, N.; Kwok, J.B.J.; Schofield, P.R.; Andreadis, A.; Snowden, J.; Craufurd, D.; Neary, D.; Owen, F.; Oostra, B.A.; Hardy, J.; Goate, A.; van Swieten, J.; Mann, D.; Lynch, T.; Heutink, P. (1998). Association of Missense and 5'-Splice-Site Mutations in Tau with the Inherited Dementia FTDP-17. Nature, 393, 702-705.

Janus, C. *et. al*. (2000), Nature 408: 979 - 982.

Kas, H.S. (1997) J Microencapsul 14: 689-711

Leon, J.; Cheng, C.K.; Neumann, P.J. (1998). Alzheimer's Disease Care: Costs and Potential Savings. Health Affairs, 17(6), 206-216.

Lippa C. F. et al. (1998) Ab-42 deposition precedes other changes in PS-1 Alzheimer's disease. Lancet 352, 1117-1118

Luo, J.-J.; Wallace, W.; Riccioni, T.; Ingram, D.K.; Roth, G.S.; Kusiak, J.W. (1999). Death of PC12 Cells and Hippocampal Neurons Induced by Adenoviral-Mediated FAD Human Amyloid Precursor Protein Gene Expression. Journal of Neuroscience Research, 55(5), 629-642.

Naruse, S.; Thinakaran, G.; Luo, J.-J.; Kusiak, J.W.; Tomita, T.; Iwatsubo, T.; Qian, X.; Ginty, D.D.; Price, D.L.; Borchelt, D.R.; Wong, P.C.; Sisodia, S.S. (1998). Effects of PS1 Deficiency on Membrane Protein Trafficking in Neurons. Neuron, 21(5), 1213-1231.

National Institute on Aging Progress Report on Alzheimer's Disease, 1999, NIH Publication No. 99-4664.

Pietrobon, P.J. (1995), Pharm Biotechnol. 6: 347-61Poorkaj, P.; Bird, T.D.; Wijsman, E.; Nemens, E.; Garruto, R. M.; Anderson, L.; Andreadis, A.; Wiederhold, W.C.; Raskind, M.; Schellenberg, G.D. (1998). Tau Is a Candidate Gene for Chromosome 17 Frontotemporal Dementia. Annals of Neurology, 43, 815-825.

Schenk, D.; Barbour, R.; Dunn, W.; Gordon, G.; Grajeda, H.; Guido, T.; Hu, K.; Huang, J.; Johnson-Wood, K.; Khan, K.; Kholodenko, D.; Lee, M.; Liao, Z.; Lieberburg, I.; Motter, R.; Mutter, L.; Soriano, F.; Shopp, G.; Vasquez, N.; Vandevert, C.; Walker, S.; Wogulis, M.; Yednock, T.; Games, D.; Seubert, P. (1999). Immunization with A-beta Attenuates Alzheimer's Disease-Like Pathology in the PDAPP Mouse. Nature, 400(6740), 173-177.

Shekunov, B. *et. al.* (1999), J. Crystal Growth 198/199: 1345 - 1351.

Spillantini, M.G.; Murrell, J.R.; Goedert, M.; Farlow, M.R.; Klug, A.; Ghetti, B. (1998). Mutation in the Tau Gene in Familial Multiple System Tauopathy with Presenile Dementia. Proceedings of the National Academy of Sciences U.S.A., 95(13), 7737-7741.

Strittmatter, W.J.; Saunders, A.M.; Schmechel, D.; Pericak-Vance, M.; Enghild, J.; Salvesen, G.S.; Roses, A.D. (1993). Apolipoprotein E: High-Avidity Binding to Aβ and Increased Frequency of Type 4 Allele in Late-Onset Familial Alzheimer Disease. Proceedings of the National Academy of Sciences U.S.A., 90,1977-1981.

Vidal, R.; Frangione, B.; Rostagno, A.; Mead, S.; Revesz, T.; Plant, G.; Ghiso, J. (1999). A Stop-Codon Mutation in the BRI Gene Associated with Familial British Dementia. Nature, 399: 776-781.

Zheng H. (1996) "Mice deficient for the amyloid precursor protein gene. Ann. N Y Acad. *Sci*., 777, 421-426.

York, P. (1999), PSTT 11: 430-440

## Claims

**1.** Use of an agent selected from

a) at least one analogue of an animal's autologous Aβ (beta amyloid) or APP (amyloid precursor protein) polypeptide wherein is introduced at least one isolated foreign T helper epitope ($T_H$ epitope) by means of amino acid insertion, addition, deletion, or substitution, wherein the foreign $T_H$ epitope is free from D-amino acids and is introduced into the autologous Aβ or APP as schematically shown for the P2 and P30 epitopes in Fig. 1,

b) a nucleic acid sequence encoding at least one analogue defined in a), and

c) a non-pathooenic microorganism or virus which carries the nucleic acid fragment defined in b),

for the preparation of a medicament containing the agent for treating and/or preventing and/or ameliorating Alzheimer's disease or other diseases and conditions **characterized by** Aβ deposits in said animal.

**2.** The use according to claim 1, wherein the introduction has as a result that a substantial fraction of B-cell epitopes of Aβ or APP are preserved and that

- at least one first moiety is introduced which effects targeting of the analogue to an antigen presenting cell (APC) or a B-lymphocyte, and/or
- at least one second moiety is introduced which stimulates the immune system, and/or
- at least one third moiety is introduced which optimizes presentation of the analogue to the immune system.

**3.** The use according to claim 2, wherein the analogue is modified by introduction as side groups, by covalent or non-covalent binding to suitable chemical groups in the Aβ, APP, or a subsequence thereof, of the first and/or of the second and/or of the third moiety.

**4.** The use according to any one of the preceding claims, wherein the analogue includes a duplication of at least one B-cell epitope of Aβ or APP and/or an introduction of a hapten.

**5.** The use according to any one of the preceding claims, wherein the foreign T-cell epitope is immunodominant in the animal.

**6.** The use according to any one of the preceding claims, wherein the foreign T-cell epitope is promiscuous, such as a foreign T-cell epitope which is selected from a natural promiscuous T-cell epitope and an artificial MHC-II binding peptide sequence.

7. The use according to claim 6, wherein the natural T-cell epitope is selected from a Tetanus toxoid epitope such as P2 or P30, a diphtheria toxoid epitope, an influenza virus hemagluttinin epitope, and a *P. falciparum* CS epitope.

8. The use according to any one of claims 2-7, wherein the first moiety is a substantially specific binding partner for a B-lymphocyte specific surface antigen or for an APC specific surface antigen such as a hapten or a carbohydrate for which there is a receptor on the B-lymphocyte or the APC.

9. The use according to any one of claims 2-8, wherein the second moiety is selected from a cytokine such as interferon $\gamma$ (IFN-$\gamma$) or an effective part thereof, FIt3L or an effective part thereof, interleukin 1 (IL-1) or an effective part thereof, interleukin 2 (IL-2) or an effective part thereof, interleukin 4 (IL-4) or an effective part thereof, interleukin 6 (IL-6) or an effective part thereof, interleukin 12 (IL-12) or an effective part thereof, interleukin 13 (IL-13) or an effective part thereof, interleukin 15 (IL-15) or an effective part thereof, and granulocyte-macroph age colony stimulating factor (GM-CSF) or an effective part thereof; a hormone; and a heat-shock protein such as HSP70 or an effective part thereof, HSP90 or an effective part thereof, HSC70 or an effective part thereof, GRP94 or an effective part thereof, and calreticulin (CRT) or an effective part thereof.

10. The use according to any one of claims 2-9, wherein the third moiety is of lipid nature, such as a palmitoyl group, a myristyl group, a famesyl group, a geranyl-geranyl group, a GPI-anchor, and an N-acyl diglyceride group, or wherein the third moiety is a polyhydroxypolymer such as a polysaccharide.

11. The use according to any one of the preceding claims, wherein the autologous A$\beta$ or APP has been modified so as to preserve B-cell epitopes which are not exposed to the extracellular phase when present in a cell-bound form of the autologous APP.

12. The use according to claim 11, wherein the A$\beta$ or APP has been modified so as to lack at least one B-cell epitope which is exposed to the extracellular phase when present in a cell-bound form of the autologous APP.

13. The use according to any of the preceding claims which comprises a substitution of at least one amino acid sequence within the autologous A$\beta$ or APP with an amino add sequence of equal or different length which gives rise to a foreign $T_H$ epitope in the analogue.

14. The use according to any one of the preceding claims, wherein the analogue comprises the amino acid sequence corresponding to amino acids 672-714 in SEQ ID NO: 2, wherein is inserted an amino add sequence which gives rise to a foreign $T_H$ epitope in the analogue, or wherein the analogue comprises an amino acid sequence corresponding to amino acids 672-714 of SEQ ID NO: 2, wherein at least one amino add sequence is substituted by an amino acid sequence of equal or different length so as to give rise to a foreign $T_H$ epitope.

15. The use according to any one of the preceding claims, wherein at least two copies of the analogue are covalently of non-covalently linked to a carrier molecule capable of effecting presentation of multiple copies of antigenic determinants.

16. The use according to any one of the preceding claims, wherein the analogue has been formulated with an adjuvant which facilitates breaking of autotolerance to autoantigens.

17. The use according to any one of the preceding claims, wherein an effective amount of the analogue is administered to the animal via a route selected from the parenteral route such as the intracutaneous, the subcutaneous, and the intramuscular routes; the peritoneal route; the oral route; the buccal route; the sublinqual route; the epidural route; the spinal route; the anal route; and the intracranial route.

18. The use according to claim 17, wherein the effective amount is between 0.5 $\mu$g and 2,000 $\mu$g of the analogue.

19. The use according to daim 17 or 18, wherein the analogue is contained in a virtual lymph node (VLN) device.

20. The use according to any one of claims 1-14, wherein the down-regulation comprises introdudng nucleic acid(s) encoding the analogue into the animal's cells and thereby obtaining *in vivo* expression by the cells of the nucleic add(s) introduced.

21. The use according to claim 20, wherein the nudeic add(s) introduced is/are selected from naked DNA, DNA formulated with charged or uncharged lipids, DNA formulated in liposomes, DNA included in a viral vector, DNA formulated with a transfection-facilitating protein or polypeptide, DNA formulated with a targeting protein or polypeptide, DNA formulated with Calcium precipitating agents, DNA coupled to an inert carrier molecule, DNA encapsulated in chitin or chitosan, and DNA formulated with an adjuvant.

**22.** The use according to claim 21, wherein the nucleic acid(s) is/are contained in a VLN device.

**23.** The use according to any one of claims 18-22, which includes at least one administration/introduction per year, such as at least 2, at least 3, at least 4, at least 6, and at least 12 administrations/introductions.

**24.** The use according to any one of claims 1-14, wherein the treatment, prevention or amelioration comprises administering a non-pathogenic microorganism or virus which is carrying a nucleic acid fragment which encodes and expresses the analogue.

**25.** An analogue of an amyloidogenic polypeptid which is derived from an animal's autologous Aβ (beta amyloid) or APP (amyloid precursor protein) wherein is introduced at least one isolated foreign $T_H$ (T helper) epitope as schematically shown for the P2 and P30 epitopes in Fig. 1, so that immunization of the animal with the analogue induces production of antibodies against the amyloidogenic polypeptide.

**26.** An analogue according to claim 24, wherein the modification is as defined in any one of claims 2-14.

**27.** An immunogenic composition comprising an immunogenically effective amount of an analogue according to claim 24 or 25, the composition further comprising a pharmaceutically and immunologically acceptable carrier and/or vehicle and optionally an adjuvant.

**28.** A nucleic add fragment which encodes an analogue according to claim 24 or 25.

**29.** A vector carrying the nucleic acid fragment according to claim 27, such as a vector that is capable of autonomous replication.

**30.** The vector according to claim 28 which is selected from the group consisting of a plasmid, a phage, a cosmid, a mini-chromosome, and a virus.

**31.** The vector according to any claim 28 or 29, comprising, in the 5'→3' direction and in operable linkage, a promoter for driving expression of the nucleic add fragment according to claim 27, optionally a nucleic acid sequence encoding a leader peptide enabling secretion of or Integration into the membrane of the polypeptide fragment, the nudeic add fragment according to claim 27, and optionally a terminator.

**32.** The vector according to any one of claims 28-30 which, when introduced into a host cell, is capable or incapable of being integrated in the host cell genome.

**33.** The vector according to claim 30 or 31, wherein a promoter drives expression in a eukaryotic cell and/or in a prokaryotic cell.

**34.** A transformed cell carrying the vector of any one of claims 28-32, such as a transformed cell which is capable of replicating the nucleic acid fragment according to claim 27.

**35.** The transformed cell according to claim 33, which is a microorganism selected from a bacterium, a yeast, a protozoan, or a cell derived from a multicellular organism selected from a fungus, an insect cell such as an $S_2$ or an SF cell, a plant cell, and a mammalian cell.

**36.** The transformed cell according to claim 33 or 34, which expresses the nudeic add fragment according to claim 27, such as a transformed cell, which secretes or carries on its surface, the analogue according to claim 25 or 26.

**37.** A composition for inducing production of antibodies against Aβ or APP in an animal where these proteins are autologous, the composition comprising

- a nudeic acid fragment according to claim 27 or a vector according to any one of claims 28-32, and

- a pharmaceutically and immunologically acceptable carrier and/or vehicle and/or adjuvant.

**38.** A stable cell line which carries the vector according to any one of claims 28-32 and which expresses the nucleic acid fragment according to claim 27, and which optionally secretes or carries the analogue according to claim 24 or 25 on its surface.

**Patentansprüche**

**1.** Verwendung eines Agenzes, ausgewählt aus

a) wenigstens einem Analogon eines autologen Aβ (beta-Amyloid)- oder APP (Amyloidprecursorprotein = Amyloidvorläuferprotein)-Polypeptids eines Tiers, in das wenigstens ein isoliertes fremdes T-Helfer-Epitop ($T_H$-Epitop) mittels Aminosäureinsertion, -addition, -deletion oder -substitution eingeführt ist, wobei das fremde $T_H$-Epitop von D-Aminosäuren frei ist und in das autologe Aβ oder APP eingeführt ist, wie es für die P2- und P30-Epitope in Fig. 1 schematisch gezeigt wird,

b) einer Nucleinsäuresequenz, die für wenigstens ein in a) definiertes Analogon codiert und

c) einem nicht-pathogenen Mikroorganismus oder Virus, der/das das in b) definierte Nucleinsäurefragment trägt,

zur Herstellung eines Medikaments, das das Agens enthält, zur Behandlung und/oder Prävention und/oder Verbesserung der Alzheimer-Krankheit oder anderer Krankheiten und Zustände, die durch Aß-Ablagerungen in dem Tier **gekennzeichnet** sind.

**2.** Verwendung nach Anspruch 1, wobei die Einführung als ein Resultat hat, dass eine wesentliche Fraktion von B-Zell-Epitopen von Aβ oder APP konserviert wird und dass

- wenigstens eine erste Gruppierung eingeführt ist, die ein Targeting des Analogons auf eine Antigen-präsentierende Zelle (APC) oder einen B-Lymphozyten bewirkt und/oder

- wenigstens eine zweite Gruppierung eingeführt ist, die das Immunsystem stimuliert, und/oder

- wenigstens eine dritte Gruppierung eingeführt ist, die eine Präsentation des Analogons gegenüber dem Immunsystem optimiert.

**3.** Verwendung nach Anspruch 2, wobei das Analogon durch Einführung der ersten und/oder der zweiten und/oder der dritten Gruppierung als Seitengruppen, durch kovalente oder nicht-kovalente Bindung an geeignete chemische Gruppen im Aβ, APP oder einer Untersequenz davon modifiziert ist.

**4.** Verwendung nach einem der vorangehenden Ansprüche, wobei das Analogon eine Verdoppelung wenigstens eines B-Zell-Epitops von Aβ oder APP und/oder eine Einführung eines Haptens umfasst.

**5.** Verwendung nach einem der vorangehenden Ansprüche, wobei das fremde T-Zell-Epitop in dem Tier immundominant ist.

**6.** Verwendung nach einem der vorangehenden Ansprüche, wobei das fremde T-Zell-Epitop promiskutiv ist, zum Beispiel ein fremdes T-Zell-Epitop ist, das aus einem natürlichen promiskutiven T-Zell-Epitop und einer künstlichen MHC-II-Bindungspeptidsequenz ausgewählt ist.

**7.** Verwendung nach Anspruch 6, wobei das natürliche T-Zell-Epitop aus einem Tetanus-Toxoid-Epitop, zum Beispiel P2 oder P30, einem Diphterie-Toxoid-Epitop, einem Influenza-Virus-Hämagglutinin-Epitop und einem P.-falciparum-CS-Epitop ausgewählt ist.

**8.** Verwendung nach einem der Ansprüche 2-7, wobei die erste Gruppierung ein im Wesentlichen spezifischer Bindungspartner für ein B-Lymphozyt-spezifisches Oberflächenantigen oder für ein APC-spezifisches Oberflächenantigen, zum Beispiel ein Hapten oder ein Kohlenhydrat, für das es einen Rezeptor an dem B-Lymphozyt oder der APC gibt, ist.

**9.** Verwendung nach einem der Ansprüche 2-8, wobei die zweite Gruppierung aus einem Cytokin, zum Beispiel Interferon-γ (IFN-γ) oder einem wirksamen Teil davon, Flt3L oder einem wirksamen Teil davon, Interleukin 1 (IL-1) oder einem wirksamen Teil davon, Interleukin 2 (IL-2) oder einem wirksamen Teil davon, Interleukin 4 (IL-4) oder einem wirksamen Teil davon, Interleukin 6 (IL-6) oder einem wirksamen Teil davon, Interleukin 12 (IL-12) oder einem wirksamen Teil davon, Interleukin 13 (IL-13) oder einem wirksamen Teil davon, Interleukin 15 (IL-15) oder einem wirksamen Teil davon und Granulozyten-Makrophagen-Koloniestimulierendem Faktor (GM-CSF) oder einem wirksamen Teil davon; einem Hormon und einem Hitzeschockprotein, zum Beispiel HSP70 oder einem wirksamen Teil davon, HSP90 oder einem wirksamen Teil davon, HSC70 oder einem wirksamen Teil davon, GRP94 oder einem wirksamen Teil davon und Calreticulin (CRT) oder einem wirksamen Teil davon ausgewählt ist.

**10.** Verwendung nach einem der Ansprüche 2-9, wobei die dritte Gruppierung Lipidnatur hat, zum Beispiel eine Palmitoylgruppe, eine Myristylgruppe, eine Farnesylgruppe, eine Geranyl-Geranyl-Gruppe, ein GPI-Anker und eine N-Acyldiglyceridgruppe, oder wobei die dritte Gruppierung ein Polyhydroxypolymer, zum Beispiel ein Polysaccharid, ist.

**11.** Verwendung nach einem der vorangehenden Ansprüche, wobei das autologe Aβ oder APP so modifiziert wurde, dass es B-Zell-Epitope konserviert, die nicht der extrazellulären Phase ausgesetzt waren, wenn sie in zellgebundener Form des autologen APP vorliegen.

**12.** Verwendung nach Anspruch 11, wobei das Aβ oder das APP so modifiziert wurde, dass ihm wenigstens ein B-Zell-Epitop fehlt, das der extrazellulären Phase ausgesetzt ist, wenn es in zellgebundener Form des autologen APP vorliegt.

**13.** Verwendung nach einem der vorangehenden Ansprüche, die ein Substitution wenigstens einer Aminosäuresequenz innerhalb des autologen Aβ oder APP durch eine Aminosäuresequenz gleicher oder unterschiedlicher Länge, die zu einem fremden $T_H$-Epitop in dem Analogon führt, umfasst.

**14.** Verwendung nach einem der vorangehenden Ansprüche, wobei das Analogon die Aminosäuresequenz umfasst, die den Aminosäuren 672-714 in SEQ ID NO:2 entspricht, worin eine Aminosäuresequenz insertiert ist, die zu einem fremden $T_H$-Epitop in dem Analogon führt, oder wobei das Analogon eine Aminosäuresequenz umfasst, die den Aminosäuren 672-714 von SEQ ID NO:2 entspricht, worin wenigstens eine Aminosäuresequenz durch eine Aminosäuresequenz gleicher oder unterschiedlicher Länge substituiert ist, so dass ein fremdes $T_H$-Epitop gebildet wird.

**15.** Verwendung nach einem der vorangehenden Ansprüche, wobei wenigstens zwei Kopien des Analogons kovalent oder nichtkovalent an ein Trägermolekül, das zur Durchführung einer Präsentation von mehreren Kopien antigener Determinanten fähig ist, gebunden sind.

**16.** Verwendung nach einem der vorangehenden Ansprüche, wobei das Analogon mit einem Adjuvans formuliert wurde, das den Abbau von Autotoleranz gegenüber Autoantigenen erleichtert.

**17.** Verwendung nach einem der vorangehenden Ansprüche, wobei eine wirksame Menge des Analogons dem Tier über einen Weg verabreicht wird, der aus dem parenteralen Weg, zum Beispiel intrakutaner, subkutaner und intramuskulärer Weg; dem peritonealen Weg; dem oralen Weg; dem bukkalen Weg; dem sublingualen Weg; dem epiduralen Weg; dem spinalen Weg; dem analen Weg und dem intrakranialen Weg ausgewählt ist.

**18.** Verwendung nach Anspruch 17, wobei die wirksame Menge zwischen 0,5 μg und 2000 μg des Analogons liegt.

**19.** Verwendung nach Anspruch 17 oder 18, wobei das Analogon in einer virtual lymph node (VLN)-Vorrichtung enthalten ist.

**20.** Verwendung nach einem der Ansprüche 1-14, wobei die Herabregulierung Einführen von Nucleinsäure(n), die für das Analogon codiert/codieren, in die Zellen des Tiers und **dadurch** Erhalt einer in-vivo-Expression der eingeführten Nucleinsäure(n) durch die Zellen umfasst.

**21.** Verwendung nach Anspruch 20, wobei die eingeführte(n) Nucleinsäure(n) aus nackter DNA, DNA, die mit geladenen oder ungeladenen Lipiden formuliert ist, DNA, die in Liposomen formuliert ist, DNA, die in einem viralen Vektor enthalten ist, DNA, die mit einem Transfektion erleichternden Protein oder Polypeptid formuliert ist, DNA, die mit einem Targeting-Protein oder -Polypeptid formuliert ist, DNA, die mit Kalziumpräzipitierungsmitteln formuliert ist, DNA, die an ein inertes Trägermolekül gebunden ist, DNA, die in Chitin oder Chitosan eingekapselt ist, und DNA, die mit einem Adjuvans formuliert ist, ausgewählt ist/sind.

**22.** Verwendung nach Anspruch 21, wobei die Nucleinsäure(n) in einer VLN-Vorrichtung enthalten ist/sind.

**23.** Verwendung nach einem der Ansprüche 18-22, die wenigstens eine Verabreichung/Einführung pro Jahr, zum Beispiel wenigstens 2, wenigstens 3, wenigstens 4, wenigstens 6 und wenigstens 12 Verabereichungen/Einführungen, umfasst.

**24.** Verwendung nach einem der Ansprüche 1-14, wobei die Behandlung, Prävention oder Verbesserung eine Verabreichung eines nicht-pathogenen Mikroorganismus oder Virus, der/das ein Nucleinsäurefragment trägt, das für das Analogon codiert und es exprimiert, umfasst.

**25.** Analogon eines amyloidogenen Polypeptids, das von einem autologen Aβ (β-Amyloid) oder APP (Amyloidvorläuferprotein) abgeleitet ist, worin wenigstens ein isoliertes fremdes $T_H$ (T-Helfer)-Epitop eingeführt ist, wie es in Fig. 1 für die P2- und P30-Epitope schematisch dargestellt ist, so dass eine Immunisierung des Tiers mit dem Analogon die Produktion von Antikörpern gegen das amyloidogene Polypeptid induziert.

**26.** Analogon nach Anspruch 24, wobei die Modifikation in einem der Ansprüche 2-14 definiert ist.

**27.** Immunogene Zusammensetzung, die eine immunogenwirksame Menge eines Analogons nach Anspruch 24 oder 25 umfasst, wobei die Zusammensetzung außerdem einen pharmazeutisch und immunologisch akzeptablen Träger und/oder ein pharmazeutisch und immunologisch akzeptables Vehikel und gegebenenfalls ein Adjuvans umfasst.

**28.** Nucleinsäurefragment, das für ein Analogon nach Anspruch 24 oder 25 codiert.

**29.** Vektor, der das Nucleinsäurefragment nach Anspruch 27 trägt, zum Beispiel ein Vektor, der zur autonomen Replikation fähig ist.

**30.** Vektor nach Anspruch 28, der aus der Gruppe, bestehend aus einem Plasmid, einem Phagen, einem Cosmid, einem Minichromosom und einem Virus ausgewählt ist.

**31.** Vektor nach Anspruch 28 oder 29, umfassend in 5'→3'-Richtung und in funktioneller Verknüpfung einen Promotor zur Steuerung der Expression des Nucleinsäurefragments nach Anspruch 27, gegebenenfalls eine Nucleinsäuresequenz, die für ein Leaderpeptid codiert, das eine Sekretion oder Integration in die Membran des Polypeptidfragments ermöglicht, das Nucleinsäurefragment nach Anspruch 27 und gegebenenfalls einen Terminator.

**32.** Vektor nach einem der Ansprüche 28-30, der, wenn er in eine Wirtszelle eingeführt wird, zur Integration in das Wirtszellgenom fähig ist oder unfähig ist.

**33.** Vektor nach Anspruch 30 oder 31, wobei ein Promotor eine Expression in einer eukaryotischen Zelle und/oder in einer prokaryotischen Zelle steuert.

**34.** Transformierte Zelle, die den Vektor nach einem der Ansprüche 28-32 trägt, zum Beispiel eine transformierte Zelle, die fähig ist, das Nucleinsäurefragment nach Anspruch 27 zu replizieren.

**35.** Transformierte Zelle nach Anspruch 33, die ein Mikroorganismus, ausgewählt aus einem Bakterium, einer Hefe, einem Protozoon, oder eine Zelle, die aus einem vielzelligen Organismus stammt, ausgewählt aus einem Fungus, eine Insektenzelle, zum Beispiel eine $S_2$- oder SF-Zelle, eine Pflanzenzelle und eine Säugerzelle, ist.

**36.** Transformierte Zelle nach Anspruch 33 oder 34, die das Nucleinsäurefragment nach Anspruch 27 exprimiert, zum Beispiel eine transformierte Zelle, die das Analogon nach Anspruch 25 oder 26 sezerniert oder an ihrer Oberfläche trägt.

**37.** Zusammensetzung zum Induzieren der Produktion von Antikörpern gegen Aβ oder APP in einem Tier, wobei diese Proteine autolog sind, wobei die Zusammensetzung umfasst:

- ein Nucleinsäurefragment nach Anspruch 27 oder einen Vektor nach einem der Ansprüche 28-32 und
- einen pharmazeutisch und immunologisch akzeptablen Träger und/oder ein pharmazeutisch und immunologisch akzeptables Vehikel und/oder ein pharmazeutisch und immunologisch akzeptables Adjuvans.

**38.** Stabile Zelllinie, die den Vektor nach einem der Ansprüche 28-32 trägt und die das Nucleinsäurefragment nach Anspruch 27 exprimiert und die gegebenenfalls das Analogon gemäß Anspruch 24 oder 25 sezerniert oder an ihrer Oberfläche trägt.

**Revendications**

**1.** Utilisation d'un agent choisi parmi

a) au moins un analogue d'un polypeptide Aβ (amyloïde β) ou APP (protéine précurseur de l'amyloïde) dans laquelle est introduit au moins un épitope de cellules T auxiliaires (épitope $T_H$) étranger au moyen d'insertion, d'addition, de délétion ou de substitution d'acide aminé, où l'épitope $T_H$ étranger est dépourvu d'acides aminés de configuration D et est introduit dans Aβ ou APP autologue comme indiqué schématiquement pour les épitopes P2 et P30 à la Figure 1,
b) une séquence d'acide nucléique codant pour au moins un analogue défini en a), et
c) un micro-organisme ou virus non pathogène qui porte le fragment d'acide nucléique défini en b)

pour la préparation d'un médicament contenant l'agent destiné au traitement et/ou à la prévention et/ou à l'amélioration de la maladie d'Alzheimer ou d'autres maladies et affections **caractérisées par** des dépôts de Aβ chez ledit animal.

**2.** Utilisation selon la revendication 1, dans laquelle l'introduction a pour effet qu'est conservée une fraction substantielle des épitopes de cellules B de Aβ ou APP et

qu'au moins un premier fragment est introduit qui opère le ciblage de l'analogue vers une cellule présentatrice d'antigène (CPA) ou un lymphocyte B, et/ou

qu'au moins un deuxième fragment est introduit qui stimule le système immunitaire, et/ou

qu'au moins un troisième fragment est introduit qui optimise la présentation de l'analogue au système immunitaire.

**3.** Utilisation selon la revendication 2, dans laquelle l'analogue est modifié par introduction sous forme de groupes latéraux, par liaison covalente ou non covalente à des groupes chimiques appropriés dans Aβ ou APP ou une sous-séquence de ceux-ci, du premier et/ou deuxième et/ou troisième fragment.

**4.** Utilisation selon l'une quelconque des revendications précédentes, dans laquelle l'analogue comprend la duplication d'au moins un épitope de cellules B de Aβ ou APP et/ou l'introduction d'un haptène.

**5.** Utilisation selon l'une quelconque des revendications précédentes, dans laquelle l'épitope de cellules T étranger est immunodominant chez l'animal.

**6.** Utilisation selon l'une quelconque des revendications précédentes, dans laquelle l'épitope de cellules T étranger est universel, tel qu'un épitope de cellules T étranger qui est choisi parmi un épitope de cellules T universel naturel et une séquence peptidique se liant au CMH II artificielle.

**7.** Utilisation selon la revendication 6, dans laquelle l'épitope de cellules T naturel est choisi parmi un épitope de l'anatoxine tétanique tel que P2 ou P30, un épitope de l'anatoxine diphtérique, un épitope de l'hémagglutinine du virus de la grippe, et un épitope du CS de *P. falciparum*.

**8.** Utilisation l'une quelconque des revendications 2 à 7, dans laquelle le premier fragment est un partenaire de liaison sensiblement spécifique d'un antigène de surface spécifique des lymphocytes B ou d'un antigène de surface spécifique des CPA tel qu'un haptène ou un glucide pour lequel il existe un récepteur sur les CPA ou les lymphocytes B.

**9.** Utilisation selon l'une quelconque des revendications 2 à 8, dans laquelle le deuxième fragment est choisi parmi une cytokine telle que l'interféron γ (IFN γ), ou un fragment efficace de celui-ci, Flt3L ou un fragment efficace de celui-ci, l'interleukine 1 (IL-1) ou un fragment efficace de celle-ci, l'interleukine 2 (IL-2) ou un fragment efficace de celle-ci, l'interleukine 4 (IL 4) ou un fragment efficace de celle-ci, l'interleukine 6 (IL-6) ou un fragment efficace de celle-ci, l'interleukine 12 (IL-12) ou un fragment efficace de celle-ci, l'interleukine 13 (IL-13) ou un fragment efficace de celle-ci, l'interleukine 15 (IL-15) ou un fragment efficace de celle-ci, et le facteur stimulant les colonies de granulocytes-macrophages (GM-CSF) ou un fragment efficace de celui-ci, une hormone; et une protéine de choc thermique telle que HSP70 ou un fragment efficace de celle-ci, HSP90 ou un fragment efficace de celle-ci, HSC70 ou un fragment efficace de celle-ci, GRP94 ou un fragment efficace de celle-ci, et la calréticuline (CRT) ou un fragment efficace de celle-ci.

**10.** Utilisation selon l'une quelconque des revendications 2 à 9, dans laquelle le troisième fragment est de nature lipidique, telle qu'un groupe palmitoyle, un groupe myristyle, un groupe famésyle, un groupe géranyle-géranyle, un élément d'ancrage GPI, et un groupe N-acyl diglycéride ou dans laquelle le troisième fragment est un polymère polyhydroxylé tel qu'un polysaccharide.

**11.** Utilisation selon l'une quelconque des revendications précédentes, dans laquelle Aβ ou APP autologue a été modifiée de façon à conserver les épitopes de cellules B qui ne sont pas exposés à la phase extracellulaire lorsqu'ils sont présents dans une forme d'APP autologue liée aux cellules.

**12.** Utilisation selon la revendication 11, dans laquelle Aβ ou APP a été modifiée de façon à être dépourvue d'au moins un épitope de cellules B qui est exposé à la phase extracellulaire lorsqu'il est présent dans une forme liée aux cellules d'APP autologue.

**13.** Utilisation selon l'une quelconque des revendications précédentes qui comprend une substitution d'au moins une séquence d'acides aminés au sein de Aβ ou APP autologue par une séquence d'acides aminés de longueur égale ou différente qui génère un épitope $T_H$ étranger dans l'analogue.

**14.** Utilisation selon l'une quelconque des revendications précédentes, dans laquelle l'analogue comprend la séquence d'acides aminés correspondant aux acides aminés 672 - 714 dans SEQ ID N° 2 où il y a insertion d'une séquence d'acides aminés qui génère un épitope $T_H$ étranger dans l'analogue, ou dans laquelle l'analogue comprend une séquence d'acides aminés correspondant aux acides aminés 672 - 714 de SEQ ID N° 2, où est substitutuée d'au moins une séquence d'acides aminés, par une séquence d'acides aminés de longueur égale ou différente de manière à générer un épitope $T_H$ étranger.

**15.** Utilisation selon l'une quelconque des revendication précédentes, dans laquelle au moins deux copies de l'analogue sont liées de manière covalente ou non covalente à une molécule porteuse capable d'opérer la présentation de multiples copies des déterminants antigéniques.

**16.** Utilisation selon l'une quelconque des revendications précédentes, dans laquelle l'analogue a été formulé avec un adjuvant qui favorise la rupture d'autotolérance aux autoantigènes.

**17.** Utilisation selon l'une quelconque des revendications précédentes, dans laquelle une quantité efficace de l'analogue est administrée à l'animal par une voie choisie parmi la voie parentérale telle que les voies intradermique, sous-cutanée, et intramusculaire, la voie péritonéale; la voie orale ; la voie buccale ; la voie sublinguate ; la voie épidurale ; la voie spinale ; la voie anale ; et la voie intracrânienne.

**18.** Utilisation selon la revendication 17, dans laquelle la quantité efficace est comprise entre 0,5 et 2000 µg d'analogue.

**19.** Utilisation selon la revendication 17 ou 18, dans laquelle l'analogue est contenu dans un dispositif de type ganglion lymphatique virtuel (VLN).

**20.** Utilisation selon l'une quelconque des revendications 1 à 14, dans laquelle la modulation négative comprend l'introduction d'un ou de plusieurs acides nucléiques codant pour l'analogue dans les cellules animales de façon à obtenir l'expression *in vivo* par les cellules du ou des acide(s) nucléique(s) introduit(s).

**21.** Utilisation selon la revendication 20, dans laquelle le ou les acide(s) nucléique(s) introduits est/sont choisis parmi l'ADN nu, l'ADN formulé avec des lipides chargés ou non chargés, l'ADN formulé dans des liposomes, l'ADN incorporé dans un vecteur viral, l'ADN formulé avec un polypeptide ou une protéine facilitant la unsfection, l'ADN formulé avec une protéine ou un polypeptide de ciblage, l'ADN formulé avec des agents précipitant le calcium, l'ADN couplé à une molécule porteuse inerte, l'ADN encapsulé dans de la chitine ou du chitosane, et l'ADN formulé avec un adjuvant.

**22.** Utilisation selon la revendication 21, dans laquelle le ou les acide(s) nucléique(s) est/sont contenus dans un dispositif VLN.

**23.** Utilisation selon l'une quelconque des revendications 18 à 22, qui comprend au moins une administration/introduction par an, tel qu'au moins 2, au moins 3, au moins 4, au moins 6, et au moins 12 administrations/introductions

**24.** Utilisation selon l'une quelconque des revendications 1 à 14, dans laquelle le traitement, la prévention ou l'amélioration comprend l'administration d'un micro-organisme ou virus non pathogène qui porte un fragment d'acide nucléique qui code et exprime l'analogue.

**25.** Analogue d'un polypeptide amyloïdogènique qui dérive de Aβ (amyloïde β) ou APP (protéine précurseur de l'amyloïde) autologue de l'animal, dans lequel il y a introduction d'au moins un épitope $T_H$ (T auxiliaires) étranger isolé comme indiqué schématiquement pour les épitopes P2 et P30 à la Figure 1, de telle sorte que l'immunisation de l'animal avec l'analogue induit la production d'anticorps dirigés contre le polypeptide amylordogènique.

**26.** Analogue selon la revendication 24, dans lequel la modification est telle que définie dans l'une quelconque des revendications 2 à 14.

**27.** Composition immunogène comprenant une quantité immunogéniquement efficace d'un analogue selon la revendication 24 ou 25, la composition comprenant en outre un porteur et/ou véhicule immunologiquement et pharmaceutiquement acceptable et éventuellement un adjuvant.

**28.** Fragment d'acide nucléique qui code pour un analogue selon la revendication 24 ou 25.

**29.** Vecteur comprenant le fragment d'acide nucléique selon la revendication 27, tel qu'un vecteur qui est capable de subir une réplication autonome.

**30.** Vecteur selon la revendication 28, qui est choisi dans le groupe constitué d'un plasmide, d'un phage, d'un cosmide, d'un minichromosome, et d'un virus.

**31.** Vecteur selon l'une quelconque des revendications 28 ou 29, comprenant dans le sens 5' → 3' et selon un enchaînement fonctionnel : un promoteur pour diriger l'expression du fragment d'acide nucléique selon la revendication 27, éventuellement une séquence d'acide nucléique codant pour un peptide signal permettant la sécrétion de ou l'intégration dans la membrane du fragment polypeptidique, le fragment d'acide nucléique selon la revendication 27, et éventuellement un signal de terminaison.

**32.** Vecteur selon l'une quelconque des revendications 28 à 30, qui lorsqu'il est introduit dans une cellule hôte, est capable de s'intégrer dans le génome de la cellule hôte ou en est incapable.

**33.** Vecteur selon la revendication 30 ou 31, dans lequel un promoteur dirige l'expression dans une cellule eucaryote et/ou procaryote.

**34.** Cellule transformée portant le vecteur selon l'une quelconque des revendications 28 à 32, telle qu'une cellule transformée qui est capable d'effectuer la réplication du fragment d'acide nucléique selon la revendication 27.

**35.** Cellule transformée selon la revendication 33, qui est un micro-organisme choisi pamni une bactérie, une levure, un protozoaire, ou une cellule dérivée d'un organisme multicellulaire choisi entre un champignon, une cellule d'insecte telle qu'une cellule SF ou $S_2$, une cellule végétale, et une cellule mammifère.

**36.** Cellule transformée selon la revendication 33 ou 34, qui exprime le fragment d'acide nucléique selon la revendication 27, telle qu'une cellule transformée, qui sécrète ou porte sur sa surface, l'analogue selon la revendication 25 ou 26.

**37.** Composition pour induire la production d'anticorps dirigés contre Aβ ou APP chez un animal où ces protéines sont autologues, la composition comprenant :

- un fragment d'acide nucléique selon la revendication 27 ou un vecteur selon l'une quelconque des revendications 28 à 32, et
- un porteur et/ou véhicule et/ou adjuvant pharmaceutiquement et immunologiquement acceptable.

**38.** Lignée cellulaire stable qui porte le vecteur selon l'une quelconque des revendications 28 à 32 et qui exprime le fragment d'acide nucléique selon la revendication 27, et qui sécrète ou porte éventuellement sur sa surface l'analogue selon la revendication 24 ou 25.

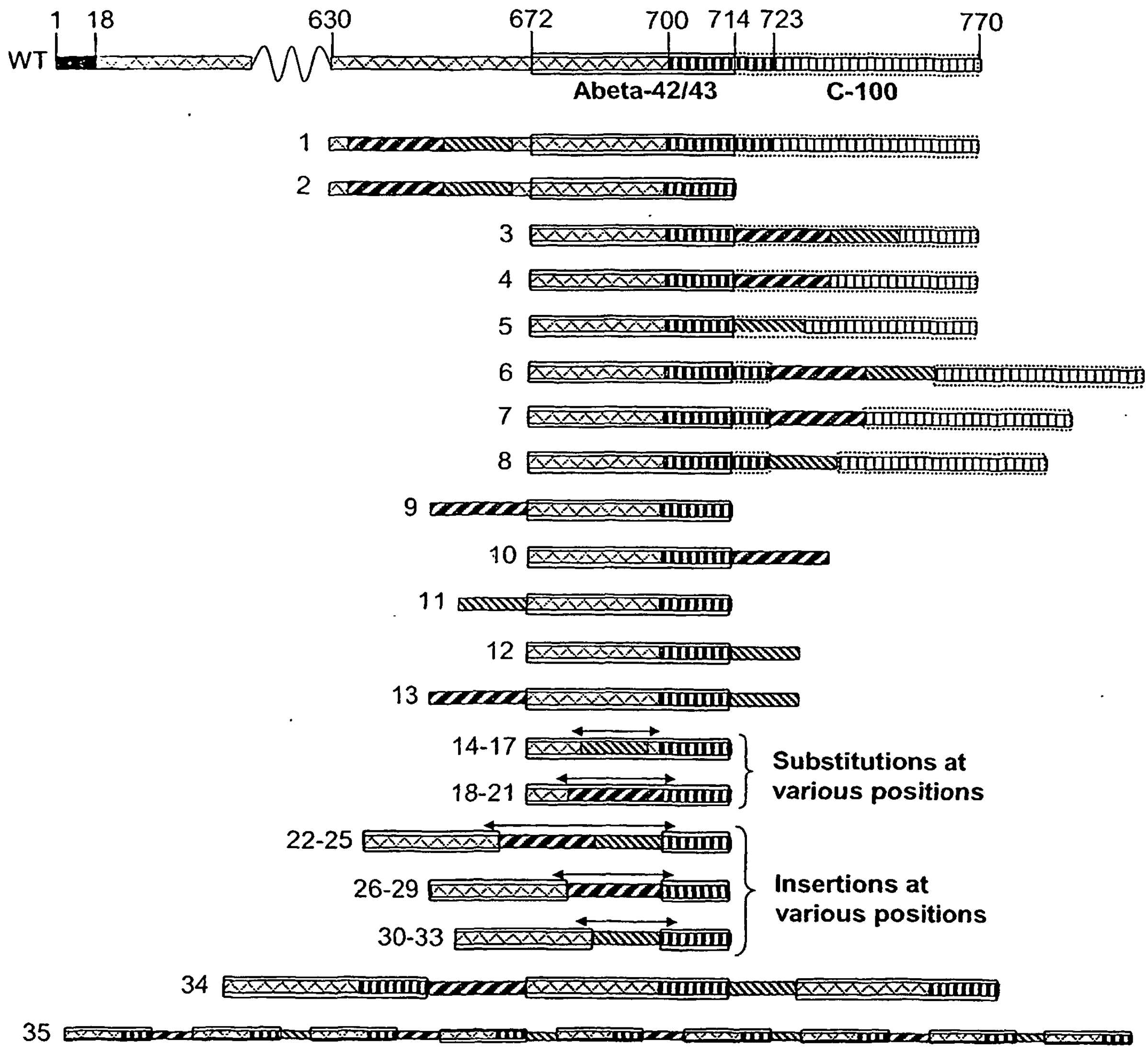
1
18
630
672
700
714
723
770
WT
Abeta-42/43
C-100
1
2
3
4
5
6
7
8
9
10
11
12
13
14-17
18-21
Substitutions at various positions
22-25
26-29
30-33
Insertions at various positions
34
35

Fig. 1